# EUROPÄISCHE PATENTSCHRIFT

(11) **EP 2 903 981 B1**
(45) Veröffentlichungstag und Bekanntmachung des Hinweises auf die Patenterteilung: **11.05.2016**
(21) Anmeldenummer: 13770916.8
(22) Anmeldetag: 01.10.2013
(51) Int. Cl.: A01N 43/707, A01N 43/82, C07D 403/12, C07D 413/12, A01N 43/713

(54) **1,2,4-TRIAZIN-3,5-DION-6-CARBOXAMIDE UND IHRE VERWENDUNG ALS HERBIZIDE**
1,2,4-TRIAZIN-3,5-DION-6-CARBOXAMIDE DERIVATIVES AND THEIR USE AS HERBICIDES
DÉRIVÉS DE 1,2,4-TRIAZIN-3,5-DION-6-CARBOXAMIDE ET L'UTILISATION COMME HERBICIDES

(30) Priorität: 04.10.2012 EP 12187187
(43) Veröffentlichungstag der Anmeldung: 12.08.2015
(73) Patentinhaber: Bayer CropScience AG, 40789 Monheim am Rhein (DE)
(72) Erfinder: BRAUN, Ralf, 76857 Ramberg (DE); WALDRAFF, Christian, 61118 Bad Vilbel (DE); DIETRICH, Hansjörg, 65835 Liederbach am Taunus (DE); GATZWEILER, Elmar, 61231 Bad Nauheim (DE); ROSINGER, Christopher Hugh, 65719 Hofheim (DE); SCHMUTZLER, Dirk, 65795 Hattersheim (DE)
(74) Vertreter: BIP Patents
(86) Internationale Anmeldenummer: PCT/EP2013/070424
(87) Internationale Veröffentlichungsnummer: WO 2014/053473

(56) Entgegenhaltungen:
- EP-A2- 1 088 819
- WO-A1-2012/002096
- WO-A1-2012/028579

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO2012/002096 A1 sind u. a. 1,2,4-Triazin-3,5-dion-6-carbonylcyclohexandione und 1,2,4-Triazin-3,5-dion-6-carbonylpyrazole als Herbizide beschrieben. Die aus diesen Schriften bekannten Verbindungen weisen jedoch nicht immer eine ausreichende herbizide Wirkung und/oder Verträglichkeit gegenüber Kulturpflanzen auf.

Es wurde nun gefunden, dass 1,2,4-Triazin-3,5-dion-6- carboxamide als Herbizide besonders gut geeignet sind.

Ein Gegenstand der vorliegenden Erfindung sind somit 1,2,4-Triazin-3,5-dion-6-carboxamide der Formel (I) oder deren Salze 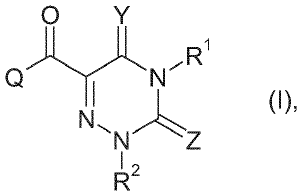 worin
R¹ bedeutet (C₁-C₁₀)-Alkyl, (C₂-C₁₀)-Alkenyl, (C₂-C₁₀)-Alkinyl, Halogen-(C₁-C₁₀)-alkyl, Halogen-(C₂-C₁₀)-alkenyl, Halogen-(C₂-C₁₀)-alkinyl, (C₃-C₁₀)-Cycloalkyl, Halogen-(C₃-C₁₀)-cycloalkyl, (C₁-₄)-Alkyl-(C₃-C₇)-cycloalkyl, (C₃-C₇)-Cycloalkyl-(C₁-C₆)-alkyl, (C₃-C₇)-Cycloalkyl-(C₃-C₇)-cycloalkyl, Halogen-(C₃-C₇)-cycloalkyl-(C₁-C₆)-alkyl, (C₁-C₆)-Alkyl-(C₃-C₇)-cycloalkyl-(C₁-C₆)-alkyl, (C₃-C₁₂)-Cycloalkenyl, Halogen-(C₃-C₁₂)-cycloalkenyl, (C₁-C₆)-Alkoxy-(C₁-C₆)-alkyl, (C₁-C₆)-Alkoxy-(C₂-C₆)-alkenyl, (C₁-C₆)-Alkyl-(C₃-C₇)-cycloalkyl, (C₁-C₆)-Alkoxy-(C₃-C₇)-cycloalkyl, (C₃-C₇)-Cycloalkoxy-(C₁-C₆)-alkyl, (C₃-C₇)-Cycloalkoxy-(C₁-C₆)-alkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, (C₁-C₆)-Alkylamino-(C₁-C₆)-alkyl, Di-(C₁-C₆)-alkylamino-(C₁-C₆)-alkyl, Halogen-(C₁-C₆)-alkylamino-(C₁-C₆)-alkyl, (C₃-C₇)-Cycloalkylamino-(C₁-C₆)-alkyl, (C₁-C₆)-Alkyl-carbonyl, Halogen-(C₁-C₆)-alkyl-carbonyl, (C₃-C₇)-Cycloalkyl-carbonyl, (C₁-C₆)-Alkoxy-carbonyl, (C₃-C₇)-Cycloalkoxy-carbonyl, (C₃-C₇)-Cycloalkyl-(C₁-C₆)-alkoxy-carbonyl, (C₁-C₆)-Alkylamino-carbonyl, Di-(C₁-C₆)-alkylamino-carbonyl, (C₃-C₇)-Cycloalkylamino-carbonyl, Cyano-(C₁-C₆)-alkyl, Hydroxy-(C₁-C₆)-alkyl, (C₃-C₇)-Cycloalkenyl-(C₁-C₆)-alkyl, Halogen-(C₁-C₆)-alkoxy-(C₁-C₆)-alkyl, (C₁-C₆)-Alkoxy-halogen-(C₁-C₆)-alkyl, Halogen-(C₁-C₆)-alkoxy-halogen-(C₁-C₆)-alkyl, Halogen-(C₃-C₇)-cycloalkoxy-(C₁-C₆)-alkyl, (C₃-C₇)-Cycloalkenyloxy-(C₁-C₆)-alkyl, Halogen-(C₃-C₇)-cycloalkenyloxy-(C₁-C₆)-alkyl, Di-(C₁-C₆)-alkoxy-(C₁-C₆)-alkyl, (C₁-C₆)-Alkoxy-(C₁-C₆)-alkyl-carbonyl, (C₁-C₆)-Alkoxy-carbonyl-(C₁-C₆)-alkyl, Halogen-(C₁-C₆)-alkoxy-carbonyl, (C₁-C₁₀)-Alkoxy, Halogen-(C₁-C10)-alkoxy, (C₃-C₁₂)-Cycloalkoxy, Halogen-(C₃-C₇)-cycloalkoxy, (C₃-C₇)-Cycloalkyl-(C₁-C₆)-alkoxy, (C₂-C₁₂)-Alkenyloxy, Halogen-(C₂-C₁₀)-alkenyloxy, (C₂-C₁₀)-Alkinyloxy, Halogen-(C₃-C₁₀)-alkinyloxy, (C₁-C₆)-alkoxy-(C₁-C₆)-alkoxy, (C₁-C₆)-Alkyl-carbonyloxy, Halogen-(C₂-C₁₂)-alkyl-carbonyloxy, (C₃-C₇)-Cycloalkyl-carbonyloxy, (C₁-C₆)-Alkyl-carbonyl-(C₁-C₆)-alkoxy, (C₁-C₁₀)-Alkylthio, Halogen-(C₁-C₁₀)-alkylthio, (C₃-C₁₂)-Cycloalkylthio, (C₁-C₁₀)-Alkylsulfinyl, Halogen-(C₁-C₁₀)-alkylsulfinyl, (C₁-C₁₀)-alkylsulfonyl, Halogen-(C₁-C₁₀)-alkylsulfonyl, (C₃-C₁₂)-Cycloalkylsulfonyl, (C₁-C₆)-Alkyl-carbonylthio, (C₁-C₆)-Alkyl(thiocarbonyl)thio, (C₃-C₁₂)-Cycloalkylsulfinyl, (C₁-C₆)-Alkylaminosulfonyl, Di-(C₁-C₆)-alkylamino-sulfonyl, (C₁-C₆)-Alkylamino, Di-(C₁-C₆)-alkylamino, Halogen-(C₁-C₆)-alkylamino, Halogen-di-(C₁-C₆)-alkylamino, (C₃-C₁₂)-Cycloalkylamino, (C₁-C₆)-Alkyl-carbonylamino, Halogen-(C₁-C₆)-alkyl-carbonylamino, (C₁-C₁₀)-Alkylsulfonylamino, Halogen-(C₁-C₁₀)-alkylsulfonylamino, (C₃-C₇)-Cycloalkyl-(C₁-C₆)-alkyl-amino, Cyano, Hydroxy, Amino, C(=O)OH, C(=O)NHOH, SO₂NH₂. SO₂NHCN, SO₂NHOH, NHCHO, oder
R¹ bedeutet Phenyl, Phenylsulfonyl, W¹-(Phenyl), W¹-(O-Phenyl), W¹-(S-Phenyl), W¹-(SO₂-Phenyl), W²-(SO₂CH₂-Phenyl) oder W²-(SCH₂-Phenyl), wobei die Phenylringe der sieben vorstehend genannten Reste jeweils s Substituenten R⁶ tragen, oder
R¹ bedeutet G, W²G oder W²OG,
R² bedeutet Wasserstoff, (C₁-C₄)-Alkyl, (C₁-C₄)-Haloalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, wobei die Phenyl-oder Heteroarylringe der vier vorstehend genannten Reste jeweils s Substituenten R⁶ tragen,
Q bedeutet einen Rest Q¹, Q², Q³ oder Q⁴ 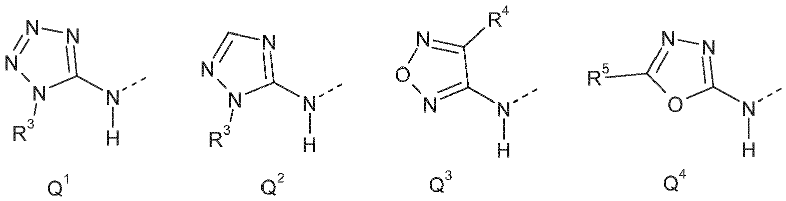
R³ bedeutet (C₁-C₈)-Alkyl, (C₂-C₈)-Alkenyl, (C₂-C₈)-Alkinyl, wobei diese Reste jeweils durch s Reste aus der Gruppe bestehend aus Halogen, Cyano, Hydroxy, Nitro, SiR¹⁰₃, PO(OR¹⁰)₂, S(O)ₙ-(C₁-C₆)-Alkyl, (C₁-C₆)-Alkoxy, Halogen-(C₁-C₆)-alkoxy, COR^{3a}, COOR^{3a}, OCOR^{3a}, NR^{3a}COR^{3a}, NR^{3a}SO₂R^{3b}, (C₃-C₆)-Cycloalkyl, Heteroaryl, Heterocyclyl oder Phenyl substituiert sind, wobei die 4 letztgenannten Reste jeweils durch p Reste aus der Gruppe bestehend aus Methyl, Ethyl, Methoxy, Trifluormethyl, Cyano und Halogen substituiert sind, und wobei Heterocyclyl n Oxogruppen trägt, oder
R³ bedeutet Phenyl, das durch p Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiert ist,
R^{3a} bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl oder Phenyl,
R^{3b} bedeutet (C₁-C₆)-Alkyl, (C₂-C₆)-Alkenyl oder (C₂-C₆)-Alkinyl, (C₃-C₆)-Cycloalkyl oder (C₃-C₆)-Cycloalkyl-(C₁-C₆)-alkyl oder Phenyl,
R⁴ bedeutet Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, (C₂-C₆)-Halogenalkenyl, (C₂-C₆)-Alkinyl, (C₂-C₆)-Alkinyloxy, (C₂-C₆)-Halogenalkinyl, (C₁-C₆)-Alkoxy-(C₁-C₆)-Alkyl, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch p Reste aus der Gruppe bestehend aus Methyl, Ethyl, Methoxy, Trifluormethyl und Halogen substituiertes Heteroaryl, Heterocyclyl oder Phenyl,
R⁵ bedeutet Wasserstoff, (C₁-C₆)-Alkyl, (C₁-C₆)-Alkoxy-(C₁-C₆)-Alkyl, CH₂R^{5a}, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR⁶, NHR⁶, Methoxycarbonyl, Ethoxycarbonyl, Methoxycarbonylmethyl, Ethoxycarbonylmethyl, Methylcarbonyl, Trifluormethylcarbonyl, Dimethylamino, Acetylamino, Methylsulfenyl, Methylsulfinyl, Methylsulfonyl oder jeweils durch p Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes Heteroaryl, Heterocyclyl, Benzyl oder Phenyl,
R^{5a} bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₁-C₆)-Alkoxy, (C₃-C₆)-Cycloalkyl oder jeweils durch p Reste aus der Gruppe bestehend aus Methyl, Ethyl, Methoxy, Trifluormethyl und Halogen substituiertes Heteroaryl oder Heterocyclyl,
Y und Z bedeuten unabhängig voneinander jeweils O oder S,
W¹ bedeutet (C₁-C₁₀)-Alkylen, (C₂-C₆)-Alkenylen oder (C₂-C₆)-Alkinylen,
W² bedeutet (C₁-C₁₀)-Alkylen,
G bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl oder Heterocyclyl, wobei Heterocyclyl n Oxogruppen trägt,
R⁶ bedeutet Halogen, Cyano, Hydroxy, Amino, Nitro, -CHO, -C(=O)OH, -C(=O)NH₂, -C(=S)NH₂, -C(=O)NHCN, -C(=O)NHOH, -SH, -SO₂NH₂, -SO₂NHCN, -SO₂NHOH, -OCN, -SCN, -SF₅, (C₁-C₆)-Alkyl, (C₂-C₆)-Alkenyl, (C₂-C₆)-Alkinyl, Halogen-(C₁-C₆)-alkyl, Halogen-(C₂-C₆)-alkenyl, Halogen-(C₂-C₆)-alkinyl, (C₃-C₈)-Cycloalkyl, Halogen-(C₃-C₈)-cycloalkyl, (C₁-C₆)-Alkyl-(C₃-C₇)-cycloalkyl, (C₃-C₇)-Cycloalkyl-(C₁-C₆)-alkyl, (C₃-C₈)-Cycloalkenyl, Halogen-(C₃-C₈)-cycloalkenyl, (C₁-C₆)-Alkoxy-(C₁-C₆)-alkyl, (C₃-C₇)-Cycloalkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₈)-Alkylsulfinyl-(C₁-C₆)-alkyl, (C₁-C₈)-Alkoxy-halogen-(C₁-C₆)-alkyl, Cyano-(C₁-C₆)-alkyl, Hydroxy-(C₁-C₆)-alkyl, (C₁-C₆)-Alkoxy, Halogen-(C₁-C₆)-alkoxy, (C₃-C₈)-Cycloalkoxy, Halogen-(C₃-C₈)-cycloalkoxy, (C₃-C₈)-Cycloalkyl-(C₁-C₆)-alkoxy, (C₂-C₆)-Alkenyloxy, Halogen-(C₂-C₆)-alkenyloxy, (C₁-C₆)-Alkoxy-(C₁-C₆)-alkoxy, (C₂-C₆)-Alkylcarbonyloxy, (C₁-C₆)-Alkylthio, Halogen-(C₁-C₆)-alkylthio, (C₃-C₈)-Cycloalkylthio, (C₁-C₆)-Alkylsulfinyl, Halogen-(C₁-C₆)-alkylsulfinyl, (C₁-C₆)-Alkylsulfonyl, Halogen-(C₁-C₆)-alkylsulfonyl, (C₃-C₈)-Cycloalkylsulfonyl, (C₁-C₆)-Alkylamino, Di-(C₁-C₆)-alkylamino, Halogen-(C₁-C₆)-alkylamino, Halogen-di-(C₁-C₈)-alkylamino oder (C₃-C₈)-Cycloalkylamino, oder zwei vicinale Reste R⁶ bilden eine (C₃-C₆)-Alkylengruppe, in der p Kohlenstoffatome durch Sauerstoff, Schwefel oder Stickstoff ersetzt sind, und in der ein Kohlenstoffatom t Oxogruppen trägt,
n bedeutet 0, 1 oder 2,
p bedeutet 0, 1, 2 oder 3,
s bedeutet 0, 1, 2, 3, 4 oder 5,
t bedeutet 0 oder 1.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann. (C₁-C₁₀)-Alkylen bedeutet entsprechend eine Methylen-, Ethylen-, jeweils unverzweigte Propylen-, Butylen-, Pentylen-, Hexylen-, Heptylen-, Octylen-, Nonylen- oder Decylen-Gruppe.

Halogen steht für Fluor, Chlor, Brom oder lod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzolring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind. Die erfindungsgemäßen Verbindungen können auf Grund der Oximether-Struktur auch als geometrische Isomere (E-/Z-Isomere) auftreten. Die Erfindung betrifft auch alle E-/Z-Isomere und deren Gemische, die von der allgemeinen Formel (I) umfasst, jedoch nicht spezifisch definiert sind.

Die Verbindungen der Formel (I) können Salze bilden. Geeignete Basen sind beispielsweise organische Amine , wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R"R''']⁺, worin R bis R''' jeweils unabhängig voneinander einen organischen Rest, insbesondere Alkyl, Aryl, Aralkyl oder Alkylaryl darstellen.

Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie p-Toluolsulfonsäure, an eine basische Gruppe, wie Amino, Alkylamino, Dialkylamino, Piperidino, Morpholino oder Pyridino, Salze bilden. Diese Salze enthalten dann die konjugierte Base der Säure als Anion.

Bevorzugt sind Verbindungen der allgemeinen Formel (I), worin
R¹ bedeutet (C₁-C₁₀)-Alkyl, (C₂-C₁₀)-Alkenyl, (C₂-C₁₀)-Alkinyl, Halogen-(C₁-C₁₀)-alkyl, Halogen-(C₂-C₁₀)-alkenyl, Halogen-(C₂-C₁₀)-alkinyl, (C₃-C₁₀)-Cycloalkyl, Halogen-(C₃-C₁₀)-cycloalkyl, (C₁-C₄)-Alkyl-(C₃-C₇)-cycloalkyl, (C₃-C₇)-Cycloalkyl-(C₁-C₆)-alkyl, (C₃-C₇)-Cycloalkyl-(C₃-C₇)-cycloalkyl, Halogen-(C₃-C₇)-cycloalkyl-(C₁-C₆)-alkyl, (C₁-C₆)-Alkyl-(C₃-C₇)-cycloalkyl-(C₁-C₆)-alkyl, (C₃-C₁₂)-Cycloalkenyl, Halogen-(C₃-C₁₂)-cycloalkenyl, (C₁-C₆)-Alkoxy-(C₁-C₆)-alkyl, (C₁-C₆)-Alkoxy-(C₂-C₆)-alkenyl, (C₁-C₆)-Alkyl-(C₃-C₇)-cycloalkyl, (C₁-C₆)-Alkoxy-(C₃-C₇)-cycloalkyl, (C₃-C₇)-Cycloalkoxy-(C₁-C₆)-alkyl, (C₃-C₇)-Cycloalkoxy-(C₁-C₆)-alkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, (C₁-C₆)-Alkylamino-(C₁-C₆)-alkyl, Di-(C₁-C₆)-alkylamino-(C₁-C₆)-alkyl, Halogen-(C₁-C₆)-alkylamino-(C₁-C₆)-alkyl, (C₃-C₇)-Cycloalkylamino-(C₁-C₆)-alkyl, (C₁-C₆)-Alkyl-carbonyl, Halogen-(C₁-C₆)-alkyl-carbonyl, (C₃-C₇)-Cycloalkyl-carbonyl, (C₁-C₆)-Alkoxy-carbonyl, (C₃-C₇)-Cycloalkoxy-carbonyl, (C₃-C₇)-Cycloalkyl-(C₁-C₆)-alkoxy-carbonyl, (C₁-C₆)-Alkylamino-carbonyl, Di-(C₁-C₆)-alkylamino-carbonyl, (C₃-C₇)-Cycloalkylamino-carbonyl, Cyano-(C₁-C₆)-alkyl, Hydroxy-(C₁-C₆)-alkyl, (C₃-C₇)-Cycloalkenyl-(C₁-C₆)-alkyl, Halogen-(C₁-C₆)-alkoxy-(C₁-C₆)-alkyl, (C₁-C₆)-Alkoxy-halogen-(C₁-C₆)-alkyl, Halogen-(C₁-C₆)-alkoxy-halogen-(C₁-C₆)-alkyl, Halogen-(C₃-C₇)-cycloalkoxy-(C₁-C₆)-alkyl, (C₃-C₇)-Cycloalkenyloxy-(C₁-C₆)-alkyl, Halogen-(C₃-C₇)-cycloalkenyloxy-(C₁-C₆)-alkyl, Di-(C₁-C₆)-alkoxy-(C₁-C₆)-alkyl, (C₁-C₆)-Alkoxy-(C₁-C₆)-alkyl-carbonyl, (C₁-C₆)-Alkoxy-carbonyl-(C₁-C₆)-alkyl, Halogen-(C₁-C₆)-alkoxy-carbonyl, (C₁-C₁₀)-Alkoxy, Halogen-(C₁-C₁₀)-alkoxy, (C₃-C₁₂)-Cycloalkoxy, Halogen-(C₃-C₇)-cycloalkoxy, (C₃-C₇)-Cycloalkyl-(C₁-C₆)-alkoxy, (C₂-C₁₂)-Alkenyloxy, Halogen-(C₂-C₁₀)-alkenyloxy, (C₂-C₁₀)-Alkinyloxy, Halogen-(C₃-C₁₀)-alkinyloxy, (C₁-C₆)-alkoxy-(C₁-C₆)-alkoxy, (C₁-C₆)-Alkyl-carbonyloxy, Halogen-(C₂-C₂)-alkyl-carbonyloxy, (C₃-C₇)-Cycloalkyl-carbonyloxy, (C₁-C₆)-Alkyl-carbonyl-(C₁-C₆)-alkoxy, (C₁-C₁₀)-Alkylthio, Halogen-(C₁-C₁₀)-alkylthio, (C₃-C₁₂)-Cycloalkylthio, (C₁-C₁₀)-Alkylsulfinyl, Halogen-(C₁-C₁₀)-alkylsulfinyl, (C₁-C₁₀)-alkylsulfonyl, Halogen-(C₁-C₁₀)-alkylsulfonyl, (C₃-C₁₂)-Cycloalkylsulfonyl, (C₁-C₆)-Alkyl-carbonylthio, (C₁-C₆)-Alkyl(thiocarbonyl)thio, (C₃-C₁₂)-Cycloalkylsulfinyl, (C₁-C₆)-Alkylaminosulfonyl, Di-(C₁-C₆)-alkylamino-sulfonyl, (C₁-C₆)-Alkylamino, Di-(C₁-C₆)-alkylamino, Halogen-(C₁-C₆)-alkylamino, Halogen-di-(C₁-C₆)-alkylamino, (C₃-C₁₂)-Cycloalkylamino, (C₁-C₆)-Alkyl-carbonylamino, Halogen-(C₁-C₆)-alkyl-carbonylamino, (C₁-C₁₀)-Alkylsulfonylamino, Halogen-(C₁-C₁₀)-alkylsulfonylamino, (C₃-C₇)-Cycloalkyl-(C₁-C₆)-alkyl-amino, Cyano, Hydroxy, Amino, C(=O)OH, C(=O)NHOH, SO₂NH₂. SO₂NHCN, SO₂NHOH, NHCHO, oder
R¹ bedeutet Phenyl, Phenylsulfonyl, W¹-(Phenyl), W¹-(O-Phenyl), W¹-(S-Phenyl), W¹-(SO₂-Phenyl), W²-(SO₂CH₂-Phenyl) oder W²-(SCH₂-Phenyl), wobei die Phenylringe der sieben vorstehend genannten Reste jeweils s Substituenten R⁶ tragen, oder R¹ bedeutet G oder W²G,
R² bedeutet Wasserstoff, (C₁-C₄)-Alkyl, (C₁-C₄)-Haloalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, wobei die Phenyl-oder Hetarylringe der vier vorstehend genannten Reste jeweils s Substituenten R⁶ tragen,
worin Q bedeutet einen Rest Q¹, Q², Q³ oder Q⁴ 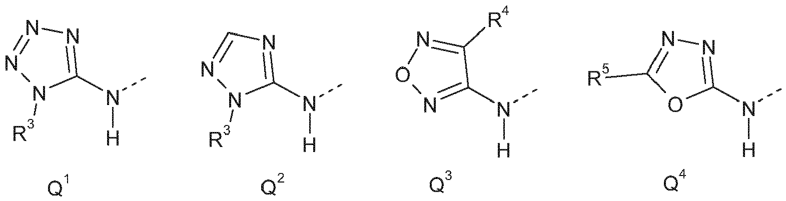 R³ bedeutet (C₁-C₈)-Alkyl, (C₂-C₈)-Alkenyl, (C₂-C₈)-Alkinyl, wobei diese Reste jeweils durch s Reste aus der Gruppe bestehend aus Halogen, Cyano, Nitro, S(O)ₙ-(C₁-C₆)-Alkyl, (C₁-C₆)-Alkoxy, Halogen-(C₁-C₆)-alkoxy, (C₃-C₆)-Cycloalkyl, Heteroaryl, Heterocyclyl oder Phenyl substituiert sind, wobei die 4 letztgenannten Reste jeweils durch p Reste aus der Gruppe bestehend aus Methyl, Ethyl, Methoxy, Trifluormethyl, Cyano und Halogen substituiert sind, und wobei Heterocyclyl n Oxogruppen trägt, oder R³ bedeutet Phenyl, das jeweils durch p Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiert ist,
R⁴ bedeutet (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano, Nitro, Methylsulfenyl, Methylsulfinyl, Methylsulfonyl, (C₁-C₄)-Alkylcarbonylamino, Benzoylamino, Methoxycarbonyl, Ethoxycarbonyl, Benzoyl, Phenoxy, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, 1,2,4-Triazol-1-yl, Pyrazol-1-yl, 2-Thiophenyl, 2-Pyridinyl, 3-Pyridinyl, 4-Pyridinyl, 1,2,4-Oxadiazol-3-yl, Benzoxazol-2-yl, 1-Ethylbenzimidazol-2-yl, Piperidin-1-yl oder jeweils durch p Reste aus der Gruppe bestehend aus Methyl, Ethyl, Methoxy, Trifluormethyl und Halogen substituiertes Phenyl,
R⁵ bedeutet Wasserstoff, (C₁-C₆)-Alkyl, (C₃-C₇)-Cycloalkyl, Halogen-(C₁-C₆)-alkyl, (C₃-C₇)-Cycloalkylmethyl, Methoxycarbonylmethyl, Ethoxycarbonylmethyl, Acetylmethyl, Methoxymethyl, Methoxyethyl, Benzyl, Pyrazin-2-yl, Furan-2-yl, Tetrahydrofuran-2-yl, Morpholin, Dimethylamino oder durch p Reste aus der Gruppe bestehend aus Methyl, Methoxy, Trifluormethyl und Halogen substituiertes Phenyl,
R⁶ bedeutet Halogen, Cyano, Hydroxy, Amino, Nitro, -CHO, -C(=O)OH, -C(=O)NH₂, -C(=S)NH₂, -C(=O)NHCN, -C(=O)NHOH, -SH, -SO₂NH₂, -SO₂NHCN, -SO₂NHOH, -OCN, -SCN, -SF₅, (C₁-C₆)-Alkyl, (C₂-C₆)-Alkenyl, (C₂-C₆)-Alkinyl, Halogen-(C₁-C₆)-alkyl, Halogen-(C₂-C₆)-alkenyl, Halogen-(C₂-C₆)-alkinyl, (C₃-C₈)-Cycloalkyl, Halogen-(C₃-C₈)-cycloalkyl, (C₁-C₆)-Alkyl-(C₃-C₇)-cycloalkyl, (C₃-C₇)-Cycloalkyl-(C₁-C₆)-alkyl, (C₃-C₈)-Cycloalkenyl, Halogen-(C₃-C₈)-cycloalkenyl, (C₁-C₆)-Alkoxy-(C₁-C₆)-alkyl, (C₃-C₇)-Cycloalkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₈)-Alkylsulfinyl-(C₁-C₆)-alkyl, (C₁-C₈)-Alkoxy-halogen-(C₁-C₆)-alkyl, Cyano-(C₁-C₆)-alkyl, Hydroxy-(C₁-C₆)-alkyl, (C₁-C₆)-Alkoxy, Halogen-(C₁-C₆)-alkoxy, (C₃-C₈)-Cycloalkoxy, Halogen-(C₃-C₈)-cycloalkoxy, (C₃-C₈)-Cycloalkyl-(C₁-C₆)-alkoxy, (C₂-C₆)-Alkenyloxy, Halogen-(C₂-C₆)-alkenyloxy, (C₁-C₆)-Alkoxy-(C₁-C₆)-alkoxy, (C₂-C₆)-Alkylcarbonyloxy, (C₁-C₆)-Alkylthio, Halogen-(C₁-C₆)-alkylthio, (C₃-C₈)-Cycloalkylthio, (C₁-C₆)-Alkylsulfinyl, Halogen-(C₁-C₆)-alkylsulfinyl, (C₁-C₆)-Alkylsulfonyl, Halogen-(C₁-C₆)-alkylsulfonyl, (C₃-C₈)-Cycloalkylsulfonyl, (C₁-C₆)-Alkylamino, Di-(C₁-C₆)-alkylamino, Halogen-(C₁-C₆)-alkylamino, Halogen-di-(C₁-C₈)-alkylamino oder (C₃-C₈)-Cycloalkylamino, oder zwei vicinale Reste R⁶ bilden eine (C₃-C₆)-Alkylengruppe, in der p Kohlenstoffatome durch Sauerstoff, Schwefel oder Stickstoff ersetzt sind, und in der ein Kohlenstoffatom t Oxogruppen trägt,
Y und Z bedeuten unabhängig voneinander jeweils O oder S,
W¹ bedeutet (C₁-C₁₀)-Alkylen, (C₂-C₆)-Alkenylen oder (C₂-C₆)-Alkinylen,
W² bedeutet (C₁-C₁₀)-Alkylen,
G bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl oder Heterocyclyl, wobei Heterocyclyl n Oxogruppen trägt,
n bedeutet 0, 1 oder 2,
p bedeutet 0, 1, 2 oder 3,
s bedeutet 0, 1, 2, 3, 4 oder 5,
t bedeutet 0 oder 1.

Besonders bevorzugt sind Verbindungen der allgemeinen Formel (I), worin
R¹ bedeutet (C₁-C₁₀)-Alkyl, (C₂-C₁₀)-Alkenyl, (C₂-C₁₀)-Alkinyl, Halogen-(C₁-C₁₀)-alkyl, Halogen-(C₂-C₁₀)-alkenyl, Halogen-(C₂-C₁₀)-alkinyl, (C₃-C₁₀)-Cycloalkyl, Halogen-(C₃-C₁₀)-cycloalkyl, (C₁-₄)-Alkyl-(C₃-C₇)-cycloalkyl, (C₃-C₇)-Cycloalkyl-(C₁-C₆)-alkyl, (C₃-C₇)-Cycloalkyl-(C₃-C₇)-cycloalkyl, Halogen-(C₃-C₇)-cycloalkyl-(C₁-C₆)-alkyl, (C₁-C₆)-Alkyl-(C₃-C₇)-cycloalkyl-(C₁-C₆)-alkyl, (C₃-C₁₂)-Cycloalkenyl, Halogen-(C₃-C₁₂)-cycloalkenyl, (C₁-C₆)-Alkoxy-(C₁-C₆)-alkyl, (C₁-C₆)-Alkoxy-(C₂-C₆)-alkenyl, (C₁-C₆)-Alkyl-(C₃-C₇)-cycloalkyl, (C₁-C₆)-Alkoxy-(C₃-C₇)-cycloalkyl, (C₃-C₇)-Cycloalkoxy-(C₁-C₆)-alkyl, (C₃-C₇)-Cycloalkoxy-(C₁-C₆)-alkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, (C₁-C₆)-Alkylamino-(C₁-C₆)-alkyl, Di-(C₁-C₆)-alkylamino-(C₁-C₆)-alkyl, Halogen-(C₁-C₆)-alkylamino-(C₁-C₆)-alkyl, (C₃-C₇)-Cycloalkylamino-(C₁-C₆)-alkyl, (C₁-C₆)-Alkyl-carbonyl, Halogen-(C₁-C₆)-alkyl-carbonyl, (C₃-C₇)-Cycloalkyl-carbonyl, (C₁-C₆)-Alkoxy-carbonyl, (C₃-C₇)-Cycloalkoxy-carbonyl, (C₃-C₇)-Cycloalkyl-(C₁-C₆)-alkoxy-carbonyl, (C₁-C₆)-Alkylamino-carbonyl, Di-(C₁-C₆)-alkylamino-carbonyl, (C₃-C₇)-Cycloalkylamino-carbonyl, Cyano-(C₁-C₆)-alkyl, Hydroxy-(C₁-C₆)-alkyl, (C₃-C₇)-Cycloalkenyl-(C₁-C₆)-alkyl, Halogen-(C₁-C₆)-alkoxy-(C₁-C₆)-alkyl, (C₁-C₆)-Alkoxy-halogen-(C₁-C₆)-alkyl, Halogen-(C₁-C₆)-alkoxy-halogen-(C₁-C₆)-alkyl, Halogen-(C₃-C₇)-cycloalkoxy-(C₁-C₆)-alkyl, (C₃-C₇)-Cycloalkenyloxy-(C₁-C₆)-alkyl, Halogen-(C₃-C₇)-cycloalkenyloxy-(C₁-C₆)-alkyl, Di-(C₁-C₆)-alkoxy-(C₁-C₆)-alkyl, (C₁-C₆)-Alkoxy-(C₁-C₆)-alkyl-carbonyl, (C₁-C₆)-Alkoxy-carbonyl-(C₁-C₆)-alkyl, Halogen-(C₁-C₆)-alkoxy-carbonyl, (C₁-C₁₀)-Alkoxy, Halogen-(C₁-C₁₀)-alkoxy, (C₃-C₁₂)-Cycloalkoxy, Halogen-(C₃-C₇)-cycloalkoxy, (C₃-C₇)-Cycloalkyl-(C₁-C₆)-alkoxy, (C₂-C₁₂)-Alkenyloxy, Halogen-(C₂-C₁₀)-alkenyloxy, (C₂-C₁₀)-Alkinyloxy, Halogen-(C₃-C₁₀)-alkinyloxy, (C₁-C₆)-alkoxy-(C₁-C₆)-alkoxy, (C₁-C₆)-Alkyl-carbonyloxy, Halogen-(C₂-C₁₂)-alkyl-carbonyloxy, (C₃-C₇)-Cycloalkyl-carbonyloxy, (C₁-C₆)-Alkyl-carbonyl-(C₁-C₆)-alkoxy, (C₁-C₁₀)-Alkylthio, Halogen-(C₁-C₁₀)-alkylthio, (C₃-C₁₂)-Cycloalkylthio, (C₁-C₁₀)-Alkylsulfinyl, Halogen-(C₁-C₁₀)-alkylsulfinyl, (C₁-C₁₀)-alkylsulfonyl, Halogen-(C₁-C₁₀)-alkylsulfonyl, (C₃-C₁₂)-Cycloalkylsulfonyl, (C₁-C₆)-Alkyl-carbonylthio, (C₁-C₆)-Alkyl(thiocarbonyl)thio, (C₃-C₁₂)-Cycloalkylsulfinyl, (C₁-C₆)-Alkylaminosulfonyl, Di-(C₁-C₆)-alkylamino-sulfonyl, (C₁-C₆)-Alkylamino, Di-(C₁-C₆)-alkylamino, Halogen-(C₁-C₆)-alkylamino, Halogen-di-(C₁-C₆)-alkylamino, (C₃-C₁₂)-Cycloalkylamino, (C₁-C₆)-Alkyl-carbonylamino, Halogen-(C₁-C₆)-alkyl-carbonylamino, (C₁-C₁₀)-Alkylsulfonylamino, Halogen-(C₁-C₁₀)-alkylsulfonylamino, (C₃-C₇)-Cycloalkyl-(C₁-C₆)-alkyl-amino, Cyano, Hydroxy, Amino, C(=O)OH, C(=O)NHOH, SO₂NH₂. SO₂NHCN, SO₂NHOH, NHCHO, oder
R¹ bedeutet Phenyl, W¹-(Phenyl), wobei die Phenylringe jeweils s Substituenten R⁶ tragen, oder
R¹ bedeutet G,
R² bedeutet (C₁-C₄)-Alkyl, (C₁-C₄)-Haloalkyl, Phenyl, Heteroaryl, wobei die Phenyl- oder Heteroarylringe der zwei vorstehend genannten Reste jeweils s Substituenten R⁶ tragen,
Q bedeutet einen Rest Q¹, Q², Q³ oder Q⁴ 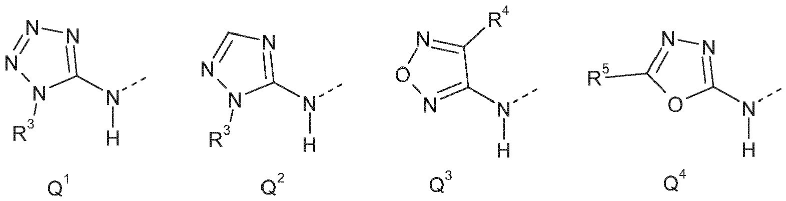 R³ bedeutet jeweils durch s Reste aus der Gruppe bestehend aus Halogen, Cyano, Nitro, S(O)ₙ-(C₁-C₆)-Alkyl, (C₁-C₆)-Alkoxy und Halogen-(C₁-C₆)-alkoxy substituiertes (C₁-C₈)-Alkyl, (C₂-C₈)-Alkenyl oder (C₂-C₈)-Alkinyl,
R⁴ bedeutet (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano, Nitro, Methylsulfenyl, Methylsulfinyl, Methylsulfonyl, (C₁-C₄)-Alkylcarbonylamino, Benzoylamino, Methoxycarbonyl, Ethoxycarbonyl, Benzoyl, Phenoxy, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, 1,2,4-Triazol-1H, 1-Pyrazol-1 H, 2-Thiophenyl, 2-Pyridinyl, 3-Pyridinyl, 4-Pyridinyl, 1,2,4-Oxadiazol-3-yl, Benzoxazol-2-yl, 1-Ethylbenzimidazol-2-yl, Piperidin-1-yl oder jeweils durch s Reste aus der Gruppe bestehend aus Methyl, Ethyl, Methoxy, Trifluormethyl und Halogen substituiertes Phenyl,
R⁵ bedeutet Wassetrstoff, (C₁-C₆)-Alkyl, (C₃-C₇)-Cycloalkyl, Halogen-(C₁-C₆)-alkyl, (C₃-C₇)-Cycloalkylmethyl, Methoxycarbonylmethyl, Ethoxycarbonylmethyl, Acetylmethyl, Methoxymethyl, Methoxyethyl, Benzyl, Pyrazin-2-yl, Furan-2-yl, Tetrahydrofuran-2-yl, Morpholin, Dimethylamino oder durch p Reste aus der Gruppe bestehend aus Methyl, Methoxy, Trifluormethyl und Halogen substituiertes Phenyl,
R⁶ bedeutet Halogen, Cyano, Nitro, (C₁-C₆)-Alkyl, (C₂-C₆)-Alkenyl, (C₂-C₆)-Alkinyl, Halogen-(C₁-C₆)-alkyl, (C₃-C₆)-Cycloalkyl, (C₁-C₆)-Alkoxy, Halogen-(C₁-C₆)-alkoxy, (C₁-C₆)-Alkylthio, (C₁-C₆)-Alkylsulfinyl, (C₁-C₆)-Alkylsulfonyl, Phenyl, Methylendioxo,
Y und Z bedeuten unabhängig voneinander jeweils O oder S,
W¹ bedeutet (C₁-C₁₀)-Alkylen,
G bedeutet jeweils durch s Reste aus der Gruppe bestehend aus Halogen, Nitro, Cyano, (C₁-C₆)-Alkyl, Halogen-(C₁-C₃)-alkyl, Cyclopropyl, S(O)ₙ-(C₁-C₃)-Alkyl, (C₁-C₄)-Alkoxy, Halogen-(C₁-C₄)-alkoxy und (C₁-C₄)-Alkoxy-(C₁-C₄)-alkyl substituiertes Heteroaryl oder Heterocyclyl, wobei Heterocyclyl n Oxogruppen trägt,
n bedeutet 0, 1 oder 2,
p bedeutet 0, 1, 2 oder 3,
s bedeutet 0, 1, 2, 3, 4 oder 5.

In allen nachfolgend genannten Formeln haben die Substituenten und Symbole, sofern nicht anders definiert, dieselbe Bedeutung wie unter Formel (I) beschrieben.

Erfindungsgemäße Verbindungen können beispielsweise gemäß Schema 1 nach den in WO 2012/028579 A1 angegebenen Methoden hergestellt werden. Diese Methoden umfassen die Umsetzung einer Säure (III) mit einem Amin Q-H und die Umsetzung eines Säurechlorids (II) mit einem Amin Q-H.

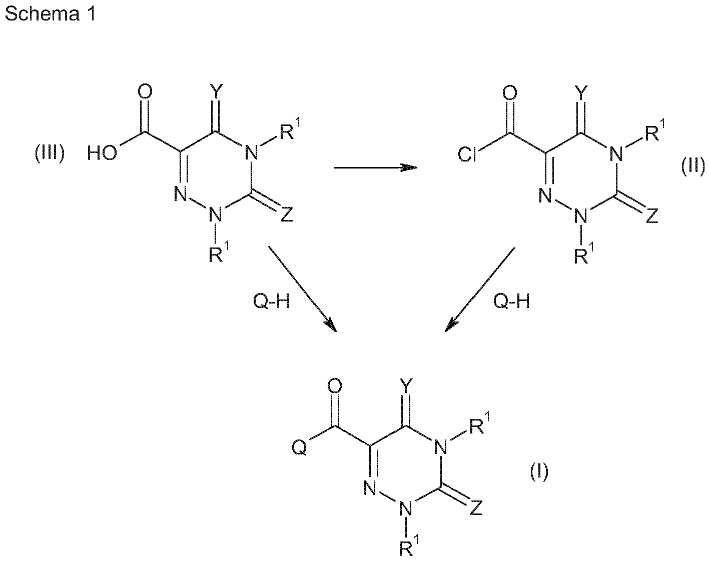

Die benötigten 1,2,4-Triazin-3,5-dion-6-carbonsäurechloride (II) beziehungsweise die ihnen zugrunde liegenden 1,2,4-Triazin-3,5-dion-6-carbonsäuren (III) können z.B. nach den in WO2012/0020961 beschriebenen Methoden hergestellt werden.

Es kann zweckmäßig sein, Reaktionsschritte in ihrer Reihenfolge zu ändern. So sind Benzoesäuren, die ein Sulfoxid tragen, nicht ohne weiteres in ihre Säurechloride zu überführen. Hier bietet sich an, zunächst auf Thioether-Stufe das Amid zu herzustellen und danach den Thioether zum Sulfoxid zu oxidieren.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### 1. Synthese von 4-Phenyl-2-methyl-N-(1-methyltetrazol-5-yl)-1,2,4-Triazin-3,5-dion-6-carbonsäureamid, (Tabellenbeispiel Nr. 1-12)

Zu 185 mg (0.75 mmol) 4-Phenyl-2-methyl-1,2,4-Triazin-3,5-dion-6-carbonsäure und 113 mg (1.15 mmol) 1-Methyl-5-aminotetrazol in 2 ml Pyridin werden bei 0°C 116 mg (0.98 mmol) Thionylchlorid gegeben. Das Gemisch wird 16 h bei Raumtemperatur (RT) gerührt. Anschließend wird mit 2N Salzsäure versetzt, das Produkt abgesaugt und mit Wasser gewaschen. Ausbeute 195 mg (79 %).

Die in nachfolgenden Tabellen aufgeführten Beispiele wurden analog oben genannten Methoden hergestellt beziehungsweise sind analog oben genannten Methoden erhältlich. Diese Verbindungen sind ganz besonders bevorzugt.

Die verwendeten Abkürzungen bedeuten:

| | | | |
|---|---|---|---|
| Et = Ethyl | Me = Methyl | nPr = n-Propyl | cPr=Cyclopropyl |
| Ph = Phenyl | | | |

**Tabelle 1: Erfindungsgemäße Verbindungen der allgemeinen Formel (I), worin Q Q¹, Y Sauerstoff, und R² Methyl bedeutet.**

| | | | | |
|---|---|---|---|---|
| 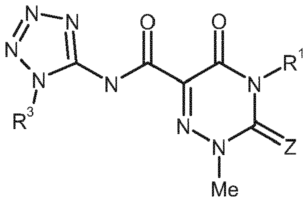 | | | | |

| Nr. | R³ | R¹ | Z | Physikalische Daten (¹H-NMR, DMSO-d_{6,} 400 MHz) |
|---|---|---|---|---|
| 1-1 | Me | Me | O | 11.32 (brs,1 H), 3.92 (s,3H), 3.65 (s,3H), 3.23 (s,3H) |
| 1-2 | Et | Me | O | 4.27 (q,2H), 3.65 (s,3H), 3.22 (s,3H), 1.43 (t,3H) |
| 1-3 | nPr | Me | O | |
| 1-4 | Me | Et | O | |
| 1-5 | Et | Et | O | |
| 1-6 | Me | nPr | O | |
| 1-7 | Me | iPr | O | |
| 1-8 | Me | cPr | O | |
| 1-9 | Me | C₂H₄OMe | O | |
| 1-10 | Me | C₂H₄OEt | O | |
| 1-11 | Me | CH₂cPr | O | |
| 1-12 | Me | Ph | O | 11.33 (brs,1H), 7.56-7.48 (m,3H), 7.33 (d,2H), 3.92 (s,3H), 3.69 (s,3H) |
| 1-13 | Me | Ph | S | |
| 1-14 | Et | Ph | O | 11.23 (brs,1H), 7.56-7.48 (m,3H), 7.33 (d,2H), 4.27 (q,2H), 3.69 (s,3H), 1.42 (t,3H) |
| 1-15 | Et | Ph | S | |
| 1-16 | nPr | Ph | O | |
| 1-17 | C₂H₄OMe | Ph | O | |
| 1-18 | Me | 2-MePh | O | |
| 1-19 | Me | 3-MePh | O | |
| 1-20 | Me | 4-MePh | O | |
| 1-21 | Me | 2-EtPh | O | |
| 1-22 | Me | 2-iPrPh | O | |
| 1-23 | Me | 2-cPrPh | O | |
| 1-24 | Me | 2-CF₃Ph | O | |
| 1-25 | Me | 3-CF₃Ph | O | |
| 1-26 | Me | 4-CF₃Ph | O | |
| 1-27 | Me | 2-CNPh | O | |
| 1-28 | Me | 2-NO₂Ph | O | |
| 1-29 | Me | 3-NO₂Ph | O | |
| 1-30 | Me | 4-NO₂Ph | O | |
| 1-31 | Me | 2-OMePh | O | |
| 1-32 | Me | 3-OMePh | O | |
| 1-33 | Me | 4-OMePh | O | |
| 1-34 | Me | 2-FPh | O | |
| 1-35 | Me | 3-FPh | O | |
| 1-36 | Me | 4-FPh | O | |
| 1-37 | Me | 2-ClPh | O | 11.45 (brs,1H), 7.71-7.69 (m,1H), 7.57-7.53 (m,3H), 3.93 (s,3H), 3.72 (s,3H) |
| 1-38 | Me | 3-ClPh | O | |
| 1-39 | Me | 4-ClPh | O | |
| 1-40 | Me | 2,4-Me₂Ph | O | |
| 1-41 | Me | 2,4-(MeO)₂Ph | O | |
| 1-42 | Me | 3,4,5-(MeO)₃Ph | O | |
| 1-43 | Me | 2-OMe-5-Cl-Ph | O | 11.40 (brs,1H), 7.53 (dd,1H), 7.42 (d,1H), 7.24 (d,1H), 3.94 (s,3H), 3.79 (s,3H), 3.72 (s,3H) |
| 1-44 | Me | 3,4-(OCH₂O)Ph | O | 11.31 (brs,1H), 7.05 (d,1H), 6.89 (d,1H), 6.80 (dd,1H), 6.12 (s,2H), 3.92 (s,3H), 3.68 (s,3H) |

**Tabelle 2: Erfindungsgemäße Verbindungen der allgemeinen Formel (I), worin Q Q², Y Sauerstoff und R² Methyl bedeutet.**

| | | | | |
|---|---|---|---|---|
| 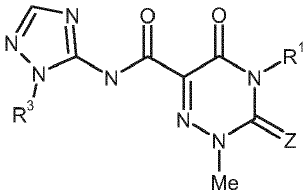 | | | | |

| Nr. | R³ | R¹ | Z | Physikalische Daten (¹H-NMR, DMSO-d_{6,} 400 MHz) |
|---|---|---|---|---|
| 2-1 | Me | Me | O | 7.91 (s,1H), 3.69 (s,3H), 3.64 (s,3H), 3.22 (s,3H) |
| 2-2 | Me | Et | O | |
| 2-3 | Me | nPr | O | |
| 2-4 | Me | iPr | O | |
| 2-5 | Me | cPr | O | |
| 2-6 | Me | C₂H₄OMe | O | |
| 2-7 | Me | C₂H₄OEt | O | |
| 2-8 | Me | CH₂cPr | O | |
| 2-9 | Me | Ph | O | 7.91 (s,1H), 7.55-7.48 (m,3H), 7.33 (d,2H), 3.69 (s,3H), 3.67 (s,3H) |
| 2-10 | Me | Ph | S | |
| 2-11 | Me | 2-MePh | O | |
| 2-12 | Me | 3-MePh | O | |
| 2-13 | Me | 4-MePh | O | |
| 2-14 | Me | 2-EtPh | O | |
| 2-15 | Me | 2-iPrPh | O | |
| 2-16 | Me | 2-cPrPh | O | |
| 2-17 | Me | 2-CF₃Ph | O | |
| 2-18 | Me | 3-CF₃Ph | O | |
| 2-19 | Me | 4-CF₃Ph | O | |
| 2-20 | Me | 2-CNPh | O | |
| 2-21 | Me | 2-NO₂Ph | O | |
| 2-22 | Me | 3-NO₂Ph | O | |
| 2-23 | Me | 4-NO₂Ph | O | |
| 2-24 | Me | 2-OMePh | O | |
| 2-25 | Me | 3-OMePh | O | |
| 2-26 | Me | 4-OMePh | O | |
| 2-27 | Me | 2-FPh | O | |
| 2-28 | Me | 3-FPh | O | |
| 2-29 | Me | 4-FPh | O | |
| 2-30 | Me | 2-ClPh | O | |
| 2-31 | Me | 3-ClPh | O | |
| 2-32 | Me | 4-ClPh | O | |
| 2-33 | Me | 2,4-Me₂Ph | O | |
| 2-34 | Me | 2,4-(MeO)₂Ph | O | |
| 2-35 | Me | 3,4,5-(MeO)sPh | O | |
| 2-36 | Me | 2-OMe-5-Cl-Ph | O | |
| 2-37 | Me | 3,4-(OCH₂O)Ph | O | |

**Tabelle 3: Erfindungsgemäße Verbindungen der allgemeinen Formel (I), worin Q Q³, Y Sauerstoff, und R² Methyl bedeutet.**

| | | | | |
|---|---|---|---|---|
| 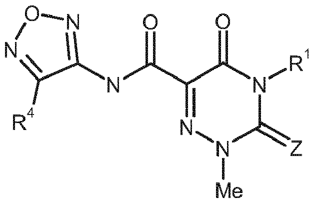 | | | | |

| Nr. | R⁴ | R¹ | Z | Physikalische Daten (¹H-NMR, DMSO-d_{6,} 400 MHz) |
|---|---|---|---|---|
| 3-1 | Me | Me | O | 11.21 (brs,1 H), 3.65 (s,3H), 3.23 (s,3H), 2.35 (s,3H) |
| 3-2 | Me | Et | O | |
| 3-3 | Me | nPr | O | |
| 3-4 | Me | iPr | O | |
| 3-5 | Me | cPr | O | |
| 3-6 | Me | C₂H₄OMe | O | |
| 3-7 | Me | C₂H₄OEt | O | |
| 3-8 | Me | CH₂cPr | O | |
| 3-9 | Me | Ph | O | 11.17 (brs,1H), 7.57-7.49 (m,3H), 7.34 m,2H), 3.69 (s,3H), 2.33 (s,3H) |
| 3-10 | Me | Ph | S | |
| 3-11 | Me | 2-MePh | O | |
| 3-12 | Me | 3-MePh | O | |
| 3-13 | Me | 4-MePh | O | |
| 3-14 | Me | 2-EtPh | O | |
| 3-15 | Me | 2-iPrPh | O | |
| 3-16 | Me | 2-cPrPh | O | |
| 3-17 | Me | 2-CF₃Ph | O | |
| 3-18 | Me | 3-CF₃Ph | O | |
| 3-19 | Me | 4-CF₃Ph | O | |
| 3-20 | Me | 2-CNPh | O | |
| 3-21 | Me | 2-NO₂Ph | O | |
| 3-22 | Me | 3-NO₂Ph | O | |
| 3-23 | Me | 4-NO₂Ph | O | |
| 3-24 | Me | 2-OMePh | O | |
| 3-25 | Me | 3-OMePh | O | |
| 3-26 | Me | 4-OMePh | O | |
| 3-27 | Me | 2-FPh | O | |
| 3-28 | Me | 3-FPh | O | |
| 3-29 | Me | 4-FPh | O | |
| 3-30 | Me | 2-ClPh | O | |
| 3-31 | Me | 3-ClPh | O | |
| 3-32 | Me | 4-ClPh | O | |
| 3-33 | Me | 2,4-Me₂Ph | O | |
| 3-34 | Me | 2,4-(MeO)₂Ph | O | |
| 3-35 | Me | 3,4,5-(MeO)₃Ph | O | |
| 3-36 | Me | 2-OMe-5-CI-Ph | O | |
| 3-37 | Me | 3,4-(OCH₂O)Ph | O | |
| 3-38 | Cl | Me | O | |
| 3-39 | Cl | Et | O | |
| 3-40 | Cl | nPr | O | |
| 3-41 | Cl | iPr | O | |
| 3-42 | Cl | cPr | O | |
| 3-43 | Cl | C₂H₄OMe | O | |
| 3-44 | Cl | C₂H₄OEt | O | |
| 3-45 | Cl | CH₂cPr | O | |
| 3-46 | Cl | Ph | O | |
| 3-47 | Cl | Ph | S | |
| 3-48 | Cl | 2-MePh | O | |
| 3-49 | Cl | 3-MePh | O | |
| 3-50 | Cl | 4-MePh | O | |
| 3-51 | Cl | 2-EtPh | O | |
| 3-52 | Cl | 2-iPrPh | O | |
| 3-53 | Cl | 2-cPrPh | O | |
| 3-54 | Cl | 2-CF₃Ph | O | |
| 3-55 | Cl | 3-CF₃Ph | O | |
| 3-56 | Cl | 4-CF₃Ph | O | |
| 3-57 | Cl | 2-CNPh | O | |
| 3-58 | Cl | 2-NO₂Ph | O | |
| 3-59 | Cl | 3-NO₂Ph | O | |
| 3-60 | Cl | 4-NO₂Ph | O | |
| 3-61 | Cl | 2-OMePh | O | |
| 3-62 | Cl | 3-OMePh | O | |
| 3-63 | Cl | 4-OMePh | O | |
| 3-64 | Cl | 2-FPh | O | |
| 3-65 | Cl | 3-FPh | O | |
| 3-66 | Cl | 4-FPh | O | |
| 3-67 | Cl | 2-ClPh | O | |
| 3-68 | Cl | 3-ClPh | O | |
| 3-69 | Cl | 4-ClPh | O | |
| 3-70 | Cl | 2,4-Me₂Ph | O | |
| 3-71 | Cl | 2,4-(MeO)₂Ph | O | |
| 3-72 | Cl | 3,4,5-(MeO)₃Ph | O | |
| 3-73 | Cl | 2-OMe-5-Cl-Ph | O | |
| 3-74 | Cl | 3,4-(OCH₂O)Ph | O | |

**Tabelle 4: Erfindungsgemäße Verbindungen der allgemeinen Formel (I), worin Q Q⁴, Y Sauerstoff, und R² Methyl bedeutet.**

| | | | | |
|---|---|---|---|---|
| 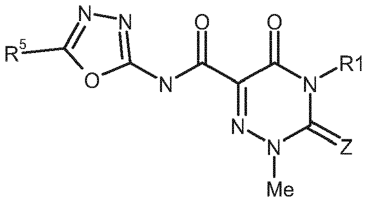 | | | | |

| Nr. | R⁵ | R¹ | Z | Physikalische Daten (¹H-NMR, DMSO-d₆, 400 MHz) |
|---|---|---|---|---|
| 4-1 | Me | Me | O | |
| 4-2 | Me | Et | O | |
| 4-3 | Me | nPr | O | |
| 4-4 | Me | iPr | O | |
| 4-5 | Me | cPr | O | |
| 4-6 | Me | C₂H₄OMe | O | |
| 4-7 | Me | C₂H₄OEt | O | |
| 4-8 | Me | CH₂cPr | O | |
| 4-9 | Me | Ph | O | 7.55-7.48 (m,3H), 7.32 (d,2H, 3.66 (s,3H)), 2.47 (s,3H) |
| 4-10 | Me | Ph | S | |
| 4-11 | Me | 2-MePh | O | |
| 4-12 | Me | 3-MePh | O | |
| 4-13 | Me | 4-MePh | O | |
| 4-14 | Me | 2-EtPh | O | |
| 4-15 | Me | 2-iPrPh | O | |
| 4-16 | Me | 2-cPrPh | O | |
| 4-17 | Me | 2-CF₃Ph | O | |
| 4-18 | Me | 3-CF₃Ph | O | |
| 4-19 | Me | 4-CF₃Ph | O | |
| 4-20 | Me | 2-CNPh | O | |
| 4-21 | Me | 2-NO₂Ph | O | |
| 4-22 | Me | 3-NO₂Ph | O | |
| 4-23 | Me | 4-NO₂Ph | O | |
| 4-24 | Me | 2-OMePh | O | |
| 4-25 | Me | 3-OMePh | O | |
| 4-26 | Me | 4-OMePh | O | |
| 4-27 | Me | 2-FPh | O | |
| 4-28 | Me | 3-FPh | O | |
| 4-29 | Me | 4-FPh | O | |
| 4-30 | Me | 2-ClPh | O | |
| 4-31 | Me | 3-ClPh | O | |
| 4-32 | Me | 4-ClPh | O | |
| 4-33 | Me | 2,4-Me₂Ph | O | |
| 4-34 | Me | 2,4-(MeO)₂Ph | O | |
| 4-35 | Me | 3,4,5-(MeO)₃Ph | O | |
| 4-36 | Me | 2-OMe-5-Cl-Ph | O | |
| 4-37 | Me | 3,4-(OCH₂O)Ph | O | |
| 4-38 | Et | Me | O | |
| 4-39 | Et | Et | O | |
| 4-40 | Et | nPr | O | |
| 4-41 | Et | iPr | O | |
| 4-42 | Et | cPr | O | |
| 4-43 | Et | C₂H₄OMe | O | |
| 4-44 | Et | C₂H₄OEt | O | |
| 4-45 | Et | CH₂cPr | O | |
| 4-46 | Et | Ph | O | |
| 4-47 | Et | Ph | S | |
| 4-48 | Et | 2-MePh | O | |
| 4-49 | Et | 3-MePh | O | |
| 4-50 | Et | 4-MePh | O | |
| 4-51 | Et | 2-EtPh | O | |
| 4-52 | Et | 2-iPrPh | O | |
| 4-53 | Et | 2-cPrPh | O | |
| 4-54 | Et | 2-CF₃Ph | O | |
| 4-55 | Et | 3-CF₃Ph | O | |
| 4-56 | Et | 4-CF₃Ph | O | |
| 4-57 | Et | 2-CNPh | O | |
| 4-58 | Et | 2-NO₂Ph | O | |
| 4-59 | Et | 3-NO₂Ph | O | |
| 4-60 | Et | 4-NO₂Ph | O | |
| 4-61 | Et | 2-OMePh | O | |
| 4-62 | Et | 3-OMePh | O | |
| 4-63 | Et | 4-OMePh | O | |
| 4-64 | Et | 2-FPh | O | |
| 4-65 | Et | 3-FPh | O | |
| 4-66 | Et | 4-FPh | O | |
| 4-67 | Et | 2-ClPh | O | |
| 4-68 | Et | 3-ClPh | O | |
| 4-69 | Et | 4-ClPh | O | |
| 4-70 | Et | 2,4-Me₂Ph | O | |
| 4-71 | Et | 2,4-(MeO)₂Ph | O | |
| 4-72 | Et | 3,4,5-(MeO)₃Ph | O | |
| 4-73 | Et | 2-OMe-5-Cl-Ph | O | |
| 4-74 | Et | 3,4-(OCH₂O)Ph | O | |
| 4-75 | OCH₂Me | Me | O | |
| 4-76 | OCH₂Me | Et | O | |
| 4-77 | OCH₂Me | nPr | O | |
| 4-78 | OCH₂Me | iPr | O | |
| 4-79 | OCH₂Me | cPr | O | |
| 4-80 | OCH₂Me | C₂H₄OMe | O | |
| 4-81 | OCH₂Me | C₂H₄OEt | O | |
| 4-82 | OCH₂Me | CH₂cPr | O | |
| 4-83 | OCH₂Me | Ph | O | |
| 4-84 | OCH₂Me | Ph | S | |
| 4-85 | OCH₂Me | 2-MePh | O | |
| 4-86 | OCH₂Me | 3-MePh | O | |
| 4-87 | OCH₂Me | 4-MePh | O | |
| 4-88 | OCH₂Me | 2-EtPh | O | |
| 4-89 | OCH₂Me | 2-iPrPh | O | |
| 4-90 | OCH₂Me | 2-cPrPh | O | |
| 4-91 | OCH₂Me | 2-CF₃Ph | O | |
| 4-92 | OCH₂Me | 3-CF₃Ph | O | |
| 4-93 | OCH₂Me | 4-CF₃Ph | O | |
| 4-94 | OCH₂Me | 2-CNPh | O | |
| 4-95 | OCH₂Me | 2-NO₂Ph | O | |
| 4-96 | OCH₂Me | 3-NO₂Ph | O | |
| 4-97 | OCH₂Me | 4-NO₂Ph | O | |
| 4-98 | OCH₂Me | 2-OMePh | O | |
| 4-99 | OCH₂Me | 3-OMePh | O | |
| 4-100 | OCH₂Me | 4-OMePh | O | |
| 4-101 | OCH₂Me | 2-FPh | O | |
| 4-102 | OCH₂Me | 3-FPh | O | |
| 4-103 | OCH₂Me | 4-FPh | O | |
| 4-104 | OCH₂Me | 2-ClPh | O | |
| 4-105 | OCH₂Me | 3-ClPh | O | |
| 4-106 | OCH₂Me | 4-ClPh | O | |
| 4-107 | OCH₂Me | 2,4-Me₂Ph | O | |
| 4-108 | OCH₂Me | 2,4-(MeO)₂Ph | O | |
| 4-109 | OCH₂Me | 3,4,5-(MeO)₃Ph | O | |
| 4-110 | OCH₂Me | 2-OMe-5-Cl-Ph | O | |
| 4-111 | OCH₂Me | 3,4-(OCH₂O)Ph | O | |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man 75 Gew.-Teile einer Verbindung der Formel (I) und/oder deren Salze, 10 Gew.-Teile ligninsulfonsaures Calcium, 5 Gew.-Teile Natriumlaurylsulfat, 3 Gew.-Teile Polyvinylalkohol und 7 Gew.-Teile Kaolin mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gew.-Teile einer Verbindung der Formel (I) und/oder deren Salze, 5 Gew.-Teile 2,2'-dinaphthylmethan-6,6'-disulfonsaures Natrium 2 Gew.-Teile oleoylmethyltaurinsaures Natrium, 1 Gew.-Teil Polyvinylalkohol, 17 Gew.-Teile Calciumcarbonat und 50 Gew.-Teile Wasser auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-1 und 1-12 bei einer Aufwandmenge von 320 g/ha eine mindestens 80%-ige Wirkung gegen Avena fatua, Cyperus serotinus, Echinochloa crus galli, Abutilon theophrasti, Amaranthus retroflexus, Stellaria media und Viola tricolor.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-1 und 1-12 bei einer Aufwandmenge von 80 g/ha eine mindestens 80%-ige Wirkung gegen Echinochloa crus galli, Abutilon theophrasti, Amaranthus retroflexus und Veronica persica.

## Patentansprüche

1. 1,2,4-Triazin-3,5-dion-6-carboxamide der Formel (I) oder deren Salze 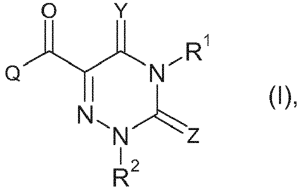 worin die Substituenten folgende Bedeutungen haben
R¹ bedeutet (C₁-C₁₀)-Alkyl, (C₂-C₁₀)-Alkenyl, (C₂-C₁₀)-Alkinyl, Halogen-(C₁-C₁₀)-alkyl, Halogen-(C₂-C₁₀)-alkenyl, Halogen-(C₂-C₁₀)-alkinyl, (C₃-C₁₀)-Cycloalkyl, Halogen-(C₃-C₁₀)-cycloalkyl, (C₁-C₄)-Alkyl-(C₃-C₇)-cycloalkyl, (C₃-C₇)-Cycloalkyl-(C₁-C₆)-alkyl, (C₃-C₇)-Cycloalkyl-(C₃-C₇)-cycloalkyl, Halogen-(C₃-C₇)-cycloalkyl-(C₁-C₆)-alkyl, (C₁-C₆)-Alkyl-(C₃-C₇)-cycloalkyl-(C₁-C₆)-alkyl, (C₃-C₁₂)-Cycloalkenyl, Halogen-(C₃-C₁₂)-cycloalkenyl, (C₁-C₆)-Alkoxy-(C₁-C₆)-alkyl, (C₁-C₆)-Alkoxy-(C₂-C₆)-alkenyl, (C₁-C₆)-Alkyl-(C₃-C₇)-cycloalkyl, (C₁-C₆)-Alkoxy-(C₃-C₇)-cycloalkyl, (C₃-C₇)-Cycloalkoxy-(C₁-C₆)-alkyl, (C₃-C₇)-Cycloalkoxy-(C₁-C₆)-alkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, (C₁-C₆)-Alkylamino-(C₁-C₆)-alkyl, Di-(C₁-C₆)-alkylamino-(C₁-C₆)-alkyl, Halogen-(C₁-C₆)-alkylamino-(C₁-C₆)-alkyl, (C₃-C₇)-Cycloalkylamino-(C₁-C₆)-alkyl, (C₁-C₆)-Alkyl-carbonyl, Halogen-(C₁-C₆)-alkyl-carbonyl, (C₃-C₇)-Cycloalkyl-carbonyl, (C₁-C₆)-Alkoxy-carbonyl, (C₃-C₇)-Cycloalkoxy-carbonyl, (C₃-C₇)-Cycloalkyl-(C₁-C₆)-alkoxy-carbonyl, (C₁-C₆)-Alkylamino-carbonyl, Di-(C₁-C₆)-alkylamino-carbonyl, (C₃-C₇)-Cycloalkylamino-carbonyl, Cyano-(C₁-C₆)-alkyl, Hydroxy-(C₁-C₆)-alkyl, (C₃-C₇)-Cycloalkenyl-(C₁-C₆)-alkyl, Halogen-(C₁-C₆)-alkoxy-(C₁-C₆)-alkyl, (C₁-C₆)-Alkoxy-halogen-(C₁-C₆)-alkyl, Halogen-(C₁-C₆)-alkoxy-halogen-(C₁-C₆)-alkyl, Halogen-(C₃-C₇)-cycloalkoxy-(C₁-C₆)-alkyl, (C₃-C₇)-Cycloalkenyloxy-(C₁-C₆)-alkyl, Halogen-(C₃-C₇)-cycloalkenyloxy-(C₁-C₆)-alkyl, Di-(C₁-C₆)-alkoxy-(C₁-C₆)-alkyl, (C₁-C₆)-Alkoxy-(C₁-C₆)-alkyl-carbonyl, (C₁-C₆)-Alkoxy-carbonyl-(C₁-C₆)-alkyl, Halogen-(C₁-C₆)-alkoxy-carbonyl, (C₁-C₁₀)-Alkoxy, Halogen-(C₁-C₁₀)-alkoxy, (C₃-C₁₂)-Cycloalkoxy, Halogen-(C₃-C₇)-cycloalkoxy, (C₃-C₇)-Cycloalkyl-(C₁-C₆)-alkoxy, (C₂-C₁₂)-Alkenyloxy, Halogen-(C₂-C₁₀)-alkenyloxy, (C₂-C₁₀)-Alkinyloxy, Halogen-(C₃-C₁₀)-alkinyloxy, (C₁-C₆)-alkoxy-(C₁-C₆)-alkoxy, (C₁-C₆)-Alkyl-carbonyloxy, Halogen-(C₂-C₁₂)-alkyl-carbonyloxy, (C₃-C₇)-Cycloalkyl-carbonyloxy, (C₁-C₆)-Alkyl-carbonyl-(C₁-C₆)-alkoxy, (C₁-C₁₀)-Alkylthio, Halogen-(C₁-C₁₀)-alkylthio, (C₃-C₁₂)-Cycloalkylthio, (C₁-C₁₀)-Alkylsulfinyl, Halogen-(C₁-C₁₀)-alkylsulfinyl, (C₁-C₁₀)-alkylsulfonyl, Halogen-(C₁-C₁₀)-alkylsulfonyl, (C₃-C₁₂)-Cycloalkylsulfonyl, (C₁-C₆)-Alkyl-carbonylthio, (C₁-C₆)-Alkyl(thiocarbonyl)thio, (C₃-C₁₂)-Cycloalkylsulfinyl, (C₁-C₆)-Alkylaminosulfonyl, Di-(C₁-C₆)-alkylamino-sulfonyl, (C₁-C₆)-Alkylamino, Di-(C₁-C₆)-alkylamino, Halogen-(C₁-C₆)-alkylamino, Halogen-di-(C₁-C₆)-alkylamino, (C₃-C₁₂)-Cycloalkylamino, (C₁-C₆)-Alkyl-carbonylamino, Halogen-(C₁-C₆)-alkyl-carbonylamino, (C₁-C₁₀)-Alkylsulfonylamino, Halogen-(C₁-C₁₀)-alkylsulfonylamino, (C₃-C₇)-Cycloalkyl-(C₁-C₆)-alkyl-amino, Cyano, Hydroxy, Amino, C(=O)OH, C(=O)NHOH, SO₂NH₂. SO₂NHCN, SO₂NHOH, NHCHO, oder
R¹ bedeutet Phenyl, Phenylsulfonyl, W¹-(Phenyl), W¹-(O-Phenyl), W¹-(S-Phenyl), W¹-(SO₂-Phenyl), W²-(SO₂CH₂-Phenyl) oder W²-(SCH₂-Phenyl), wobei die Phenylringe der sieben vorstehend genannten Reste jeweils s Substituenten R⁶ tragen, oder
R¹ bedeutet G, W²G oder W²OG,
R² bedeutet Wasserstoff, (C₁-C₄)-Alkyl, (C₁-C₄)-Haloalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, wobei die Phenyl- oder Heteroarylringe der vier vorstehend genannten Reste jeweils s Substituenten R⁶ tragen,
Q bedeutet einen Rest Q¹, Q², Q³ oder Q⁴ 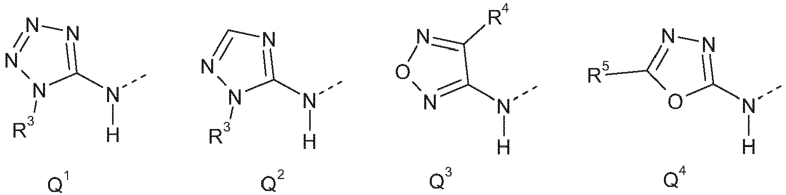
R³ bedeutet (C₁-C₈)-Alkyl, (C₂-C₈)-Alkenyl, (C₂-C₈)-Alkinyl, wobei diese Reste jeweils durch s Reste aus der Gruppe bestehend aus Halogen, Cyano, Hydroxy, Nitro, SiR¹⁰₃, PO(OR¹⁰)₂, S(O)ₙ-(C₁-C₆)-Alkyl, (C₁-C₆)-Alkoxy, Halogen-(C₁-C₆)-alkoxy, COR^{3a}, COOR^{3a}, OCOR^{3a}, NR^{3a}COR^{3a}, NR^{3a}SO₂R^{3b}, (C₃-C₆)-Cycloalkyl, Heteroaryl, Heterocyclyl oder Phenyl substituiert sind, wobei die 4 letztgenannten Reste jeweils durch p Reste aus der Gruppe bestehend aus Methyl, Ethyl, Methoxy, Trifluormethyl, Cyano und Halogen substituiert sind, und wobei Heterocyclyl n Oxogruppen trägt, oder
R³ bedeutet Phenyl, das durch p Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiert ist,
R^{3a} bedeutet Wasserstoff, (C₁-C₆)-Alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl oder Phenyl,
R^{3b} bedeutet (C₁-C₆)-Alkyl, (C₂-C₆)-Alkenyl oder (C₂-C₆)-Alkinyl, (C₃-C₆)-Cycloalkyl oder (C₃-C₆)-Cycloalkyl-(C₁-C₆)-alkyl oder Phenyl,
R⁴ bedeutet Wasserstoff, (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, (C₂-C₆)-Alkenyl, (C₂-C₆)-Alkenyloxy, (C₂-C₆)-Halogenalkenyl, (C₂-C₆)-Alkinyl, (C₂-C₆)-Alkinyloxy, (C₂-C₆)-Halogenalkinyl, (C₁-C₆)-Alkoxy-(C₁-C₆)-Alkyl, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch p Reste aus der Gruppe bestehend aus Methyl, Ethyl, Methoxy, Trifluormethyl und Halogen substituiertes Heteroaryl, Heterocyclyl oder Phenyl,
R⁵ bedeutet Wasserstoff, (C₁-C₆)-Alkyl, (C₁-C₆)-Alkoxy-(C₁-C₆)-Alkyl, CH₂R^{5a}, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, OR⁶, NHR⁶, Methoxycarbonyl, Ethoxycarbonyl, Methoxycarbonylmethyl, Ethoxycarbonylmethyl, Methylcarbonyl, Trifluormethylcarbonyl, Dimethylamino, Acetylamino, Methylsulfenyl, Methylsulfinyl, Methylsulfonyl oder jeweils durch p Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiertes Heteroaryl, Heterocyclyl, Benzyl oder Phenyl,
R^{5a} bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, (C₁-C₆)-Alkoxy, (C₃-C₆)-Cycloalkyl oder jeweils durch p Reste aus der Gruppe bestehend aus Methyl, Ethyl, Methoxy, Trifluormethyl und Halogen substituiertes Heteroaryl oder Heterocyclyl,
Y und Z bedeuten unabhängig voneinander jeweils O oder S,
W¹ bedeutet (C₁-C₁₀)-Alkylen, (C₂-C₆)-Alkenylen oder (C₂-C₆)-Alkinylen,
W² bedeutet (C₁-C₁₀)-Alkylen,
G bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl oder Heterocyclyl, wobei Heterocyclyl n Oxogruppen trägt,
R⁶ bedeutet Halogen, Cyano, Hydroxy, Amino, Nitro, -CHO, -C(=O)OH, -C(=O)NH₂, -C(=S)NH₂, -C(=O)NHCN, -C(=O)NHOH, -SH, -SO₂NH₂, -SO₂NHCN, -SO₂NHOH, -OCN, -SCN, -SF₅, (C₁-C₆)-Alkyl, (C₂-C₆)-Alkenyl, (C₂-C₆)-Alkinyl, Halogen-(C₁-C₆)-alkyl, Halogen-(C₂-C₆)-alkenyl, Halogen-(C₂-C₆)-alkinyl, (C₃-C₈)-Cycloalkyl, Halogen-(C₃-C₈)-CyCloalkyl, (C₁-C₆)-Alkyl-(C₃-C₇)-cycloalkyl, (C₃-C₇)-Cycloalkyl-(C₁-C₆)-alkyl, (C₃-C₈)-Cycloalkenyl, Halogen-(C₃-C₈)-cycloalkenyl, (C₁-C₆)-Alkoxy-(C₁-C₆)-alkyl, (C₃-C₇)-Cycloalkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₈)-Alkylsulfinyl-(C₁-C₆)-alkyl, (C₁-C₈)-Alkoxy-halogen-(C₁-C₆)-alkyl, Cyano-(C₁-C₆)-alkyl, Hydroxy-(C₁-C₆)-alkyl, (C₁-C₆)-Alkoxy, Halogen-(C₁-C₆)-alkoxy, (C₃-C₈)-Cycloalkoxy, Halogen-(C₃-C₈)-cycloalkoxy, (C₃-C₈)-Cycloalkyl-(C₁-C₆)-alkoxy, (C₂-C₆)-Alkenyloxy, Halogen-(C₂-C₆)-alkenyloxy, (C₁-C₆)-Alkoxy-(C₁-C₆)-alkoxy, (C₂-C₆)-Alkylcarbonyloxy, (C₁-C₆)-Alkylthio, Halogen-(C₁-C₆)-alkylthio, (C₃-C₈)-Cycloalkylthio, (C₁-C₆)-Alkylsulfinyl, Halogen-(C₁-C₆)-alkylsulfinyl, (C₁-C₆)-Alkylsulfonyl, Halogen-(C₁-C₆)-alkylsulfonyl, (C₃-C₈)-Cycloalkylsulfonyl, (C₁-C₆)-Alkylamino, Di-(C₁-C₆)-alkylamino, Halogen-(C₁-C₆)-alkylamino, Halogen-di-(C₁-C₈)-alkylamino oder (C₃-C₈)-Cycloalkylamino, oder zwei vicinale Reste R⁶ bilden eine (C₃-C₆)-Alkylengruppe, in der p Kohlenstoffatome durch Sauerstoff, Schwefel oder Stickstoff ersetzt sind, und in der ein Kohlenstoffatom t Oxogruppen trägt,
n bedeutet 0, 1 oder 2,
p bedeutet 0, 1, 2 oder 3,
s bedeutet 0, 1, 2, 3, 4 oder 5,
t bedeutet 0 oder 1.

2. 1,2,4-Triazin-3,5-dion-6-carboxamide der Formel (I) oder deren Salze nach Anspruch 1, worin
R¹ bedeutet (C₁-C₁₀)-Alkyl, (C₂-C₁₀)-Alkenyl, (C₂-C₁₀)-Alkinyl, Halogen-(C₁-C₁₀)-alkyl, Halogen-(C₂-C₁₀)-alkenyl, Halogen-(C₂-C₁₀)-alkinyl, (C₃-C₁₀)-Cycloalkyl, Halogen-(C₃-C₁₀)-cycloalkyl, (C₁-C₄)-Alkyl-(C₃-C₇)-cycloalkyl, (C₃-C₇)-Cycloalkyl-(C₁-C₆)-alkyl, (C₃-C₇)-Cycloalkyl-(Cs-C₇)-cycloalkyl, Halogen-(C₃-C₇)-cycloalkyl-(C₁-C₆)-alkyl, (C₁-C₆)-Alkyl-(C₃-C₇)-cycloalkyl-(C₁-C₆)-alkyl, (C₃-C₁₂)-Cycloalkenyl, Halogen-(C₃-C₁₂)-cycloalkenyl, (C₁-C₆)-Alkoxy-(C₁-C₆)-alkyl, (C₁-C₆)-Alkoxy-(C₂-C₆)-alkenyl, (C₁-C₆)-Alkyl-(C₃-C₇)-cycloalkyl, (C₁-C₆)-Alkoxy-(C₃-C₇)-cycloalkyl, (C₃-C₇)-Cycloalkoxy-(C₁-C₆)-alkyl, (C₃-C₇)-Cycloalkoxy-(C₁-C₆)-alkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, (C₁-C₆)-Alkylamino-(C₁-C₆)-alkyl, Di-(C₁-C₆)-alkylamino-(C₁-C₆)-alkyl, Halogen-(C₁-C₆)-alkylamino-(C₁-C₆)-alkyl, (C₃-C₇)-Cycloalkylamino-(C₁-C₆)-alkyl, (C₁-C₆)-Alkyl-carbonyl, Halogen-(C₁-C₆)-alkyl-carbonyl, (C₃-C₇)-Cycloalkyl-carbonyl, (C₁-C₆)-Alkoxy-carbonyl, (C₃-C₇)-Cycloalkoxy-carbonyl, (C₃-C₇)-Cycloalkyl-(C₁-C₆)-alkoxy-carbonyl, (C₁-C₆)-Alkylamino-carbonyl, Di-(C₁-C₆)-alkylamino-carbonyl, (C₃-C₇)-Cycloalkylamino-carbonyl, Cyano-(C₁-C₆)-alkyl, Hydroxy-(C₁-C₆)-alkyl, (C₃-C₇)-Cycloalkenyl-(C₁-C₆)-alkyl, Halogen-(C₁-C₆)-alkoxy-(C₁-C₆)-alkyl, (C₁-C₆)-Alkoxy-halogen-(C₁-C₆)-alkyl, Halogen-(C₁-C₆)-alkoxy-halogen-(C₁-C₆)-alkyl, Halogen-(C₃-C₇)-cycloalkoxy-(C₁-C₆)-alkyl, (C₃-C₇)-Cycloalkenyloxy-(C₁-C₆)-alkyl, Halogen-(C₃-C₇)-cycloalkenyloxy-(C₁-C₆)-alkyl, Di-(C₁-C₆)-alkoxy-(C₁-C₆)-alkyl, (C₁-C₆)-Alkoxy-(C₁-C₆)-alkyl-carbonyl, (C₁-C₆)-Alkoxy-carbonyl-(C₁-C₆)-alkyl, Halogen-(C₁-C₆)-alkoxy-carbonyl, (C₁-C₁₀)-Alkoxy, Halogen-(C₁-C₁₀)-alkoxy, (C₃-C₁₂)-Cycloalkoxy, Halogen-(C₃-C₇)-cycloalkoxy, (C₃-C₇)-Cycloalkyl-(C₁-C₆)-alkoxy, (C₂-C₁₂)-Alkenyloxy, Halogen-(C₂-C₁₀)-alkenyloxy, (C₂-C₁₀)-Alkinyloxy, Halogen-(C₃-C₁₀)-alkinyloxy, (C₁-C₆)-alkoxy-(C₁-C₆)-alkoxy, (C₁-C₆)-Alkyl-carbonyloxy, Halogen-(C₂-C₁₂)-alkyl-carbonyloxy, (C₃-C₇)-Cycloalkyl-carbonyloxy, (C₁-C₆)-Alkyl-carbonyl-(C₁-C₆)-alkoxy, (C₁-C₁₀)-Alkylthio, Halogen-(C₁-C₁₀)-alkylthio, (C₃-C₁₂)-Cycloalkylthio, (C₁-C₁₀)-Alkylsulfinyl, Halogen-(C₁-C₁₀)-alkylsulfinyl, (C₁-C₁₀)-alkylsulfonyl, Halogen-(C₁-C₁₀)-alkylsulfonyl, (C₃-C₁₂)-Cycloalkylsulfonyl, (C₁-C₆)-Alkyl-carbonylthio, (C₁-C₆)-Alkyl(thiocarbonyl)thio, (C₃-C₁₂)-Cycloalkylsulfinyl, (C₁-C₆)-Alkylaminosulfonyl, Di-(C₁-C₆)-alkylamino-sulfonyl, (C₁-C₆)-Alkylamino, Di-(C₁-C₆)-alkylamino, Halogen-(C₁-C₆)-alkylamino, Halogen-di-(C₁-C₆)-alkylamino, (C₃-C₁₂)-Cycloalkylamino, (C₁-C₆)-Alkyl-carbonylamino, Halogen-(C₁-C₆)-alkyl-carbonylamino, (C₁-C₁₀)-Alkylsulfonylamino, Halogen-(C₁-C₁₀)-alkylsulfonylamino, (C₃-C₇)-Cycloalkyl-(C₁-C₆)-alkyl-amino, Cyano, Hydroxy, Amino, C(=O)OH, C(=O)NHOH, SO₂NH₂. SO₂NHCN, SO₂NHOH, NHCHO, oder
R¹ bedeutet Phenyl, Phenylsulfonyl, W¹-(Phenyl), W¹-(O-Phenyl), W¹-(S-Phenyl), W¹-(SO₂-Phenyl), W²-(SPO₂CH₂-Phenyl) oder W²-(SCH₂-Phenyl), wobei die Phenylringe der sieben vorstehend genannten Reste jeweils s Substituenten R⁶ tragen, oder
R¹ bedeutet G oder W²G,
R² bedeutet Wasserstoff, (C₁-C₄)-Alkyl, (C₁-C₄)-Haloalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, wobei die Phenyl- oder Hetarylringe der vier vorstehend genannten Reste jeweils s Substituenten R⁶ tragen,
worin Q bedeutet einen Rest Q¹, Q², Q³ oder Q⁴ 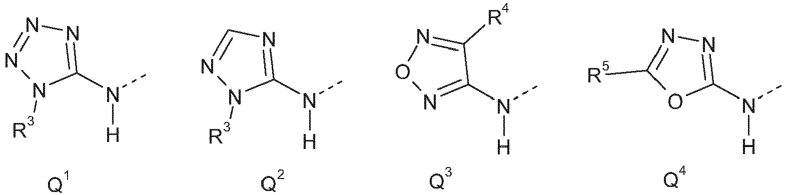 R³ bedeutet (C₁-C₈)-Alkyl, (C₂-C₈)-Alkenyl, (C₂-C₈)-Alkinyl, wobei diese Reste jeweils durch s Reste aus der Gruppe bestehend aus Halogen, Cyano, Nitro, S(O)ₙ-(C₁-C₆)-Alkyl, (C₁-C₆)-Alkoxy, Halogen-(C₁-C₆)-alkoxy, (C₃-C₆)-Cycloalkyl, Heteroaryl, Heterocyclyl oder Phenyl substituiert sind, wobei die 4 letztgenannten Reste jeweils durch p Reste aus der Gruppe bestehend aus Methyl, Ethyl, Methoxy, Trifluormethyl, Cyano und Halogen substituiert sind, und wobei Heterocyclyl n Oxogruppen trägt, oder R³ bedeutet Phenyl, das jeweils durch p Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl substituiert ist,
R⁴ bedeutet (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano, Nitro, Methylsulfenyl, Methylsulfinyl, Methylsulfonyl, (C₁-C₄)-Alkylcarbonylamino, Benzoylamino, Methoxycarbonyl, Ethoxycarbonyl, Benzoyl, Phenoxy, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, 1,2,4-Triazol-1-yl, Pyrazol-1-yl, 2-Thiophenyl, 2-Pyridinyl, 3-Pyridinyl, 4-Pyridinyl, 1,2,4-Oxadiazol-3-yl, Benzoxazol-2-yl, 1-Ethylbenzimidazol-2-yl, Piperidin-1-yl oder jeweils durch p Reste aus der Gruppe bestehend aus Methyl, Ethyl, Methoxy, Trifluormethyl und Halogen substituiertes Phenyl,
R⁵ bedeutet Wasserstoff, (C₁-C₆)-Alkyl, (C₃-C₇)-Cycloalkyl, Halogen-(C₁-C₆)-alkyl, (C₃-C₇)-Cycloalkylmethyl, Methoxycarbonylmethyl, Ethoxycarbonylmethyl, Acetylmethyl, Methoxymethyl, Methoxyethyl, Benzyl, Pyrazin-2-yl, Furan-2-yl, Tetrahydrofuran-2-yl, Morpholin, Dimethylamino oder durch p Reste aus der Gruppe bestehend aus Methyl, Methoxy, Trifluormethyl und Halogen substituiertes Phenyl,
R⁶ bedeutet Halogen, Cyano, Hydroxy, Amino, Nitro, -CHO, -C(=O)OH, -C(=O)NH₂, -C(=S)NH₂, -C(=O)NHCN, -C(=O)NHOH, -SH, -SO₂NH₂, -SO₂NHCN, -SO₂NHOH, -OCN, -SCN, -SF₅, (C₁-C₆)-Alkyl, (C₂-C₆)-Alkenyl, (C₂-C₆)-Alkinyl, Halogen-(C₁-C₆)-alkyl, Halogen-(C₂-C₆)-alkenyl, Halogen-(C₂-C₆)-alkinyl, (C₃-C₈)-Cycloalkyl, Halogen-(C₃-C₈)-cycloalkyl, (C₁-C₆)-Alkyl-(C₃-C₇)-cycloalkyl, (C₃-C₇)-Cycloalkyl-(C₁-C₆)-alkyl, (C₃-C₈)-Cycloalkenyl, Halogen-(C₃-C₈)-cycloalkenyl, (C₁-C₆)-Alkoxy-(C₁-C₆)-alkyl, (C₃-C₇)-Cycloalkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₈)-Alkylsulfinyl-(C₁-C₆)-alkyl, (C₁-C₈)-Alkoxy-halogen-(C₁-C₆)-alkyl, Cyano-(C₁-C₆)-alkyl, Hydroxy-(C₁-C₆)-alkyl, (C₁-C₆)-Alkoxy, Halogen-(C₁-C₆)-alkoxy, (C₃-C₈)-Cycloalkoxy, Halogen-(C₃-C₈)-cycloalkoxy, (C₃-C₈)-Cycloalkyl-(C₁-C₆)-alkoxy, (C₂-C₆)-Alkenyloxy, Halogen-(C₂-C₆)-alkenyloxy, (C₁-C₆)-Alkoxy-(C₁-C₆)-alkoxy, (C₂-C₆)-Alkylcarbonyloxy, (C₁-C₆)-Alkylthio, Halogen-(C₁-C₆)-alkylthio, (C₃-C₈)-Cycloalkylthio, (C₁-C₆)-Alkylsulfinyl, Halogen-(C₁-C₆)-alkylsulfinyl, (C₁-C₆)-Alkylsulfonyl, Halogen-(C₁-C₆)-alkylsulfonyl, (C₃-C₈)-Cycloalkylsulfonyl, (C₁-C₆)-Alkylamino, Di-(C₁-C₆)-alkylamino, Halogen-(C₁-C₆)-alkylamino, Halogen-di-(C₁-C₈)-alkylamino oder (C₃-C₈)-Cycloalkylamino, oder zwei vicinale Reste R⁶ bilden eine (C₃-C₆)-Alkylengruppe, in der p Kohlenstoffatome durch Sauerstoff, Schwefel oder Stickstoff ersetzt sind, und in der ein Kohlenstoffatom t Oxogruppen trägt,
Y und Z bedeuten unabhängig voneinander jeweils O oder S,
W¹ bedeutet (C₁-C₁₀)-Alkylen, (C₂-C₆)-Alkenylen oder (C₂-C₆)-Alkinylen,
W² bedeutet (C₁-C₁₀)-Alkylen,
G bedeutet jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₄)-alkyl substituiertes Heteroaryl oder Heterocyclyl, wobei Heterocyclyl n Oxogruppen trägt,
n bedeutet 0, 1 oder 2,
p bedeutet 0, 1, 2 oder 3,
s bedeutet 0, 1, 2, 3, 4 oder 5,
t bedeutet 0 oder 1.

3. 1,2,4-Triazin-3,5-dion-6-carboxamide der Formel (I) oder deren Salze nach Anspruch 1 oder 2, worin
R¹ bedeutet (C₁-C₁₀)-Alkyl, (C₂-C₁₀)-Alkenyl, (C₂-C₁₀)-Alkinyl, Halogen-(C₁-C₁₀)-alkyl, Halogen-(C₂-C₁₀)-alkenyl, Halogen-(C₂-C₁₀)-alkinyl, (C₃-C₁₀)-Cycloalkyl, Halogen-(C₃-C₁₀)-cycloalkyl, (C₁-C₄)-Alkyl-(C₃-C₇)-Cycloalkyl-(C₁-C₆)-alkyl, (C₃-C₇)-Cycloalkyl-(C₃-C₇)-cycloalkyl, Halogen-(C₃-C₇)-cycloalkyl-(C₁-C₆)-alkyl, (C₁-C₆)-Alkyl-(C₃-C₇)-cycloalkyl-(C₁-C₆)-alkyl, (C₃-C₁₂)-Cycloalkenyl, Halogen-(C₃-C₁₂)-cycloalkenyl, (C₁-C₆)-Alkoxy-(C₁-C₆)-alkyl, (C₁-C₆)-Alkoxy-(C₂-C₆)-alkenyl, (C₁-C₆)-Alkyl-(C₃-C₇)-cycloalkyl, (C₁-C₆)-Alkoxy-(C₃-C₇)-cycloalkyl, (C₃-C₇)-Cycloalkoxy-(C₁-C₆)-alkyl, (C₃-C₇)-Cycloalkoxy-(C₁-C₆)-alkoxy-(C₁-C₆)-alkyl, (C₁-C₆)-Alkoxy-(C₁-C₆)-alkoxy-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Alkylsulfonyl-(C₁-C₆)-alkyl, (C₁-C₆)-Alkylamino-(C₁-C₆)-alkyl, Di-(C₁-C₆)-alkylamino-(C₁-C₆)-alkyl, Halogen-(C₁-C₆)-alkylamino-(C₁-C₆)-alkyl, (C₃-C₇)-Cycloalkylamino-(C₁-C₆)-alkyl, (C₁-C₆)-Alkyl-carbonyl, Halogen-(C₁-C₆)-alkyl-carbonyl, (C₃-C₇)-Cycloalkyl-carbonyl, (C₁-C₆)-Alkoxy-carbonyl, (C₃-C₇)-Cycloalkoxy-carbonyl, (C₃-C₇)-Cycloalkyl-(C₁-C₆)-alkoxy-carbonyl, (C₁-C₆)-Alkylamino-carbonyl, Di-(C₁-C₆)-alkylamino-carbonyl, (C₃-C₇)-Cycloalkylamino-carbonyl, Cyano-(C₁-C₆)-alkyl, Hydroxy-(C₁-C₆)-alkyl, (C₃-C₇)-Cycloalkenyl-(C₁-C₆)-alkyl, Halogen-(C₁-C₆)-alkoxy-(C₁-C₆)-alkyl, (C₁-C₆)-Alkoxy-halogen-(C₁-C₆)-alkyl, Halogen-(C₁-C₆)-alkoxy-halogen-(C₁-C₆)-alkyl, Halogen-(C₃-C₇)-cycloalkoxy-(C₃-C₇)-alkyl, (C₃-C₇)-Cycloalkenyloxy-(C₁-C₆)-alkyl, Halogen-(C₃-C₇)-cycloalkenyloxy-(C₁-C₆)-alkyl, Di-(C₁-C₆)-alkoxy-(C₁-C₆)-alkyl, (C₁-C₆)-Alkoxy-(C₁-C₆)-alkyl-carbonyl, (C₁-C₆)-Alkoxy-carbonyl-(C₁-C₆)-alkyl, Halogen-(C₁-C₆)-alkoxy-carbonyl, (C₁-C₁₀)-Alkoxy, Halogen-(C₁-C₁₀)-alkoxy, (C₃-C₁₂)-Cycloalkoxy, Halogen-(C₃-C₇)-cycloalkoxy, (C₃-C₇)-Cycloalkyl-(C₁-C₆)-alkoxy, (C₂-C₁₂)-Alkenyloxy, Halogen-(C₂-C₁₀)-alkenyloxy, (C₂-C₁₀)-Alkinyloxy, Halogen-(C₃-C₁₀)-alkinyloxy, (C₁-C₆)-alkoxy-(C₁-C₆)-alkoxy, (C₁-C₆)-Alkyl-carbonyloxy, Halogen-(C₂-C₁₂)-alkyl-carbonyloxy, (C₃-C₇)-Cycloalkyl-carbonyloxy, (C₁-C₆)-Alkyl-carbonyl-(C₁-C₆)-alkoxy, (C₁-C₁₀)-Alkylthio, Halogen-(C₁-C₁₀)-alkylthio, (C₃-C₁₂)-Cycloalkylthio, (C₁-C₁₀)-Alkylsulfinyl, Halogen-(C₁-C₁₀)-alkylsulfinyl, (C₁-C₁₀)-alkylsulfonyl, Halogen-(C₁-C₁₀)-alkylsulfonyl, (C₃-C₁₂)-Cycloalkylsulfonyl, (C₁-C₆)-Alkyl-carbonylthio, (C₁-C₆)-Alkyl(thiocarbonyl)thio, (C₃-C₁₂)-Cycloalkylsulfinyl, (C₁-C₆)-Alkylaminosulfonyl, Di-(C₁-C₆)-alkylamino-sulfonyl, (C₁-C₆)-Alkylamino, Di-(C₁-C₆)-alkylamino, Halogen-(C₁-C₆)-alkylamino, Halogen-di-(C₁-C₆)-alkylamino, (C₃-C₁₂)-Cycloalkylamino, (C₁-C₆)-Alkyl-carbonylamino, Halogen-(C₁-C₆)-alkyl-carbonylamino, (C₁-C₁₀)-Alkylsulfonylamino, Halogen-(C₁-C₁₀)-alkylsulfonylamino, (C₃-C₇)-Cycloalkyl-(C₁-C₆)-alkyl-amino, Cyano, Hydroxy, Amino, C(=O)OH, C(=O)NHOH, SO₂NH₂. SO₂NHCN, SO₂NHOH, NHCHO, oder
R¹ bedeutet Phenyl, W¹-(Phenyl), wobei die Phenylringe jeweils s Substituenten R⁶ tragen, oder
R¹ bedeutet G,
R² bedeutet (C₁-C₄)-Alkyl, (C₁-C₄)-Haloalkyl, Phenyl, Heteroaryl, wobei die Phenyl- oder Heteroarylringe der zwei vorstehend genannten Reste jeweils s Substituenten R⁶ tragen,
Q bedeutet einen Rest Q¹, Q², Q³ oder Q⁴ 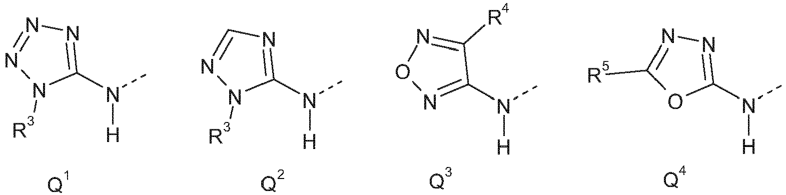 R³ bedeutet jeweils durch s Reste aus der Gruppe bestehend aus Halogen, Cyano, Nitro, S(O)ₙ-(C₁-C₆)-Alkyl, (C₁-C₆)-Alkoxy und Halogen-(C₁-C₆)-alkoxy substituiertes (C₁-C₈)-Alkyl, (C₂-C₈)-Alkenyl oder (C₂-C₈)-Alkinyl,
R⁴ bedeutet (C₁-C₆)-Alkyl, (C₃-C₇)-cyclo-Alkyl, Halogen-(C₁-C₆)-alkyl, (C₁-C₆)-Alkoxy, Halogen-(C₁-C₆)-alkoxy, Cyano, Nitro, Methylsulfenyl, Methylsulfinyl, Methylsulfonyl, (C₁-C₄)-Alkylcarbonylamino, Benzoylamino, Methoxycarbonyl, Ethoxycarbonyl, Benzoyl, Phenoxy, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, 1,2,4-Triazol-1H, 1-Pyrazol-1 H, 2-Thiophenyl, 2-Pyridinyl, 3-Pyridinyl, 4-Pyridinyl, 1,2,4-Oxadiazol-3-yl, Benzoxazol-2-yl, 1-Ethylbenzimidazol-2-yl, Piperidin-1-yl oder jeweils durch s Reste aus der Gruppe bestehend aus Methyl, Ethyl, Methoxy, Trifluormethyl und Halogen substituiertes Phenyl,
R⁵ bedeutet Wassetrstoff, (C₁-C₆)-Alkyl, (C₃-C₇)-Cycloalkyl, Halogen-(C₁-C₆)-alkyl, (C₃-C₇)-Cycloalkylmethyl, Methoxycarbonylmethyl, Ethoxycarbonylmethyl, Acetylmethyl, Methoxymethyl, Methoxyethyl, Benzyl, Pyrazin-2-yl, Furan-2-yl, Tetrahydrofuran-2-yl, Morpholin, Dimethylamino oder durch p Reste aus der Gruppe bestehend aus Methyl, Methoxy, Trifluormethyl und Halogen substituiertes Phenyl,
R⁶ bedeutet Halogen, Cyano, Nitro, (C₁-C₆)-Alkyl, (C₂-C₆)-Alkenyl, (C₂-C₆)-Alkinyl, Halogen-(C₁-C₆)-alkyl, (C₃-C₆)-Cycloalkyl, (C₁-C₆)-Alkoxy, Halogen-(C₁-C₆)-alkoxy, (C₁-C₆)-Alkylthio, (C₁-C₆)-Alkylsulfinyl, (C₁-C₆)-Alkylsulfonyl, Phenyl, Methylendioxo,
Y und Z bedeuten unabhängig voneinander jeweils O oder S,
W¹ bedeutet (C₁-C₁₀)-Alkylen,
G bedeutet jeweils durch s Reste aus der Gruppe bestehend aus Halogen, Nitro, Cyano, (C₁-C₆)-Alkyl, Halogen-(C₁-C₃)-alkyl, Cyclopropyl, S(O)ₙ-(C₁-C₃)-Alkyl, (C₁-C₄)-Alkoxy, Halogen-(C₁-C₄)-alkoxy und (C₁-C₄)-Alkoxy-(C₁-C₄)-alkyl substituiertes Heteroaryl oder Heterocyclyl, wobei Heterocyclyl n Oxogruppen trägt,
n bedeutet 0, 1 oder 2,
p bedeutet 0, 1, 2 oder 3,
s bedeutet 0, 1, 2, 3, 4 oder 5.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

7. Herbizide Mittel nach Anspruch 6 enthaltend einen Safener.

8. Herbizide Mittel nach Anspruch 7 enthaltend cyprosulfamid, cloquintocet-mexyl, mefenpyr-diethyl oder isoxadifen-ethyl.

9. Herbizide Mittel nach einem der Ansprüche 4 bis 8 enthaltend ein weiteres Herbizid.

10. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 8 auf die Pflanzen oder auf den Ort des unerwünschten Pflanzenwachstums appliziert.

11. Verwendung von Verbindungen der Formel (I) gemäß einem der Ansprüche 1 bis 3 oder von herbiziden Mitteln nach einem der Ansprüche 4 bis 8 zur Bekämpfung unerwünschter Pflanzen.

12. Verwendung nach Anspruch 11, **dadurch gekennzeichnet, daß** die Verbindungen der Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

## Claims

1. 1,2,4-Triazine-3,5-dione-6-carboxamides of the formula (I) or salts thereof 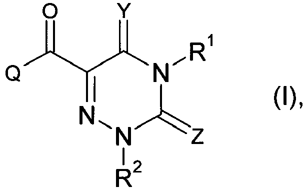 in which the substituents are defined as follows:
R¹ is (C₁-C₁₀) -alkyl, (C₂-C₁₀) -alkenyl, (C₂-C₁₀)-alkynyl, halo- (C₁-C₁₀) -alkyl, halo- (C₂-C₁₀) -alkenyl, halo-(C₂-C₁₀)-alkynyl, (C₃-C₁₀) -cycloalkyl, halo- (C₃-C₁₀)-cycloalkyl, (C₁-C₄) -alkyl- (C₃-C₇) -cycloalkyl, (C₃-C₇)-cycloalkyl- (C₁-C₆) -alkyl, (C₃-C₇)-cycloalkyl-(C₃-C₇)-cycloalkyl, halo- (C₃-C₇) -cycloalkyl- (C₁-C₆) -alkyl, (C₁-C₆) -alkyl- (C₃-C₇) -cycloalkyl- (C₁-C₆) -alkyl, (C₃-C₁₂)-cycloalkenyl, halo- (C₃-C₁₂) -cycloalkenyl, (C₁-C₆)-alkoxy-(C₁-C₆)-alkyl, (C₁-C₆)-alkoxy-(C₂-C₆)-alkenyl, (C₁-C₆)-alkyl-(C₃-C₇)-cycloalkyl, (C₁-C₆)-alkoxy-(C₃-C₇)-cycloalkyl, (C₃-C₇)-cycloalkoxy-(C₁-C₆)-alkyl, (C₃**-**C₇)-cycloalkoxy- (C₁-C₆) -alkoxy- (C₁-C₆) -alkyl, (C₁-C₆) -alkoxy-(C₁-C₆) -alkoxy- (C₁-C₆) -alkyl, (C₁-C₆) -alkylthio- (C₁-C₆)-alkyl, (C₁-C₆)-alkylsulfinyl-(C₁-C₆)-alkyl, (C₁-C₆)-alkylsulfonyl-(C₁-C₆)-alkyl, (C₁-C₆)-alkylamino-(C₁-C₆)-alkyl, di-(C₁-C₆)-alkylamino-(C₁-C₆)-alkyl, halo-(C₁-C₆)-alkylamino-(C₁-C₆)-alkyl, (C₃-C₇)-cycloalkylamino-(C₁-C₆) -alkyl, (C₁-C₆)-alkylcarbonyl, halo-(C₁-C₆)-alkylcarbonyl, (C₃-C₇)-cycloalkylcarbonyl, (C₁-C₆)-alkoxycarbonyl, (C₃-C₇)-cycloalkoxycarbonyl, (C₃-C₇)-cycloalkyl-(C₁-C₆)-alkoxycarbonyl, (C₁-C₆)-alkylaminocarbonyl, di-(C₁-C₆)-alkylaminocarbonyl, (C₃-C₇)-cycloalkylaminocarbonyl, cyano-(C₁-C₆)-alkyl, hydroxy-(C₁-C₆)-alkyl, (C₃-C₇) -cycloalkenyl-(C₁-C₆)-alkyl, halo-(C₁-C₆)-alkoxy-(C₁-C₆)-alkyl, (C₁-C₆)-alkoxyhalo-(C₁-C₆)-alkyl, halo-(C₁-C₆)-alkoxyhalo-(C₁-C₆)-alkyl, halo-(C₃-C₇)-cycloalkoxy-(C₁-C₆)-alkyl, (C₃-C₇)-cycloalkenyloxy-(C₁-C₆)-alkyl, halo-(C₃-C₇)-cycloalkenyloxy-(C₁-C₆)-alkyl, di-(C₁-C₆)-alkoxy-(C₁-C₆)-alkyl, (C₁-C₆)-alkoxy-(C₁-C₆)-alkylcarbonyl, (C₁-C₆)-alkoxycarbonyl-(C₁-C₆)-alkyl, halo-(C₁-C₆)-alkoxycarbonyl, (C₁-C₁₀)-alkoxy, halo- (C₁-C₁₀) -alkoxy, (C₃-C₁₂)-cycloalkoxy, halo-(C₃-C₇)-cycloalkoxy, (C₃-C₇)-cycloalkyl-(C₁-C₆)-alkoxy, (C₂-C₁₂)-alkenyloxy, halo-(C₂-C₁₀)-alkenyloxy, (C₂-C₁₀) -alkynyloxy, halo-(C₃-C₁₀)-alkynyloxy, (C₁-C₆)-alkoxy-(C₁-C₆)-alkoxy, (C₁-C₆)-alkylcarbonyloxy, halo-(C₂-C₁₂)-alkylcarbonyloxy, (C₃-C₇) -cycloalkylcarbonyloxy, (C₁-C₆)-alkylcarbonyl-(C₁-C₆)-alkoxy, (C₁-C₁₀)-alkylthio, halo-(C₁-C₁₀)-alkylthio, (C₃-C₁₂)-cycloalkylthio, (C₁-C₁₀)-alkylsulfinyl, halo-(C₁-C₁₀)-alkylsulfinyl, (C₁-C₁₀)-alkylsulfonyl, halo-(C₁-C₁₀)-alkylsulfonyl, (C₃-C₁₂)-cycloalkylsulfonyl, (C₁-C₆)-alkylcarbonylthio, (C₁-C₆)-alkyl(thiocarbonyl)thio, (C₃-C₁₂)-cycloalkylsulfinyl, (C₁-C₆)-alkylaminosulfonyl, di-(C₁-C₆)-alkylaminosulfonyl, (C₁-C₆)-alkylamino, di-(C₁-C₆)-alkylamino, halo-(C₁-C₆)-alkylamino, halodi-(C₁-C₆)-alkylamino, (C₃-C₁₂)-cycloalkylamino, (C₁-C₆)-alkylcarbonylamino, halo-(C₁-C₆)-alkylcarbonylamino, (C₁-C₁₀)-alkylsulfonylamino, halo-(C₁-C₁₀)-alkylsulfonylamino, (C₃-C₇)-cycloalkyl-(C₁-C₆)-alkylamino, cyano, hydroxyl, amino, C(=O)OH, C(=O)NHOH, SO₂NH₂, SO₂NHCN, SO₂NHOH, NHCHO, or
R¹ is phenyl, phenylsulfonyl, W¹-(phenyl), W¹- (O-phenyl), W¹-(S-phenyl), W¹-(SO₂-phenyl), W²-(SO₂CH₂-phenyl) or W²-(SCH₂-phenyl), where the phenyl rings of the seven aforementioned radicals each bear s R⁶ substituents, or
R¹ is G, W²G or W²OG,
R² is hydrogen, (C₁-C₄)-alkyl, (C₁-C₄)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-halocycloalkyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, where the phenyl or heteroaryl rings of the four aforementioned radicals each bear s R⁶ substituents,
Q is a Q¹, Q², Q³ or Q⁴ radical, 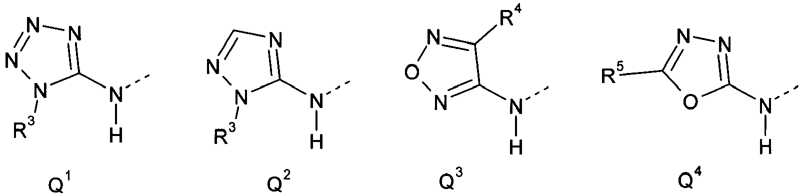
R³ is (C₁-C₈)-alkyl, (C₂-C₈)-alkenyl, (C₂-C₈-alkynyl, where these radicals are each substituted by s radicals from the group consisting of halogen, cyano, hydroxyl, nitro, SiR¹⁰₃, PO(OR¹⁰)₂, S(O)ₙ-(C₁-C₆)-alkyl, (C₁-C₆)-alkoxy, halo- (C₁-C₆) -alkoxy, COR^{3a}, COOR^{3a}, OCOR^{3a}, NR^{3a}COR^{3a}, NR^{3a}SO₂R^{3b}, (C₃-C₆-cycloalkyl, heteroaryl, heterocyclyl or phenyl, where the 4 latter radicals are each substituted by p radicals from the group consisting of methyl, ethyl, methoxy, trifluoromethyl, cyano and halogen, and where heterocyclyl bears n oxo groups, or
R³ is phenyl substituted by p radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy, halo-(C₁-C₆)-alkoxy and (C₁-C₆)-alkoxy-(C₁-C₄)-alkyl,
R^{3a} is hydrogen, (C₁-C₆)-alkyl, (C₂-C₆)-alkenyl, (C₂-C₆)-alkynyl, (C₃-C₆)-cycloalkyl, (C₃-C₆) -cycloalkyl- (C₁-C₆)-alkyl or phenyl,
R^{3b} is (C₁-C₆)-alkyl, (C₂-C₆) -alkenyl, (C₂-C₆)-alkynyl, (C₃-C₆) -cycloalkyl, (C₃-C₆)-cycloalkyl-(C₁-C₆)-alkyl or phenyl,
R⁴ is hydrogen, (C₁-C₆)-alkyl, (C₃-C₇)-cycloalkyl, halo-(C₁-C₆)-alkyl, (C₁-C₆)-alkoxy, halo-(C₁-C₆)-alkoxy, (C₂-C₆)-alkenyl, (C₂-C₆)-alkenyloxy, (C₂-C₆)-haloalkenyl, (C₂-C₆)-alkynyl, (C₂-C₆)-alkynyloxy, (C₂-C₆) -haloalkynyl, (C₁-C₆)-alkoxy-(C₁-C₆)-alkyl, cyano, nitro, methylsulfenyl, methylsulfinyl, methylsulfonyl, acetylamino, benzoylamino, methoxycarbonyl, ethoxycarbonyl, methoxycarbonylmethyl, ethoxycarbonylmethyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl, or heteroaryl, heterocyclyl or phenyl, each of which is substituted by p radicals from the group consisting of methyl, ethyl, methoxy, trifluoromethyl and halogen,
R⁵ is hydrogen, (C₁-C₆)-alkyl, (C₁-C₆)-alkoxy-(C₁-C₆)-alkyl, CH₂R^{5a}, (C₃-C₇)-cycloalkyl, halo-(C₁-C₆)-alkyl, (C₂-C₆)-alkenyl, halo-(C₂-C₆)-alkenyl, (C₂-C₆)-alkynyl, halo-(C₂-C₆)-alkynyl, OR⁶, NHR⁶, methoxycarbonyl, ethoxycarbonyl, methoxycarbonylmethyl, ethoxycarbonylmethyl, methylcarbonyl, trifluoromethylcarbonyl, dimethylamino, acetylamino, methylsulfenyl, methylsulfinyl or methylsulfonyl, or heteroaryl, heterocyclyl, benzyl or phenyl, each of which is substituted by p radicals from the group consisting of halogen, nitro, cyano, (C₁-C₆)-alkyl, halo-(C₁-C₆)-alkyl, (C₃-C₆) -cycloalkyl, S(O)ₙ-(C₁-C₆)-alkyl, (C₁-C₆)-alkoxy, halo-(C₁-C₆)-alkoxy and (C₁-C₆)-alkoxy-(C₁-C₄)-alkyl,
R⁵a is acetoxy, acetamido, N-methylacetamido, benzoyloxy, benzamido, N-methylbenzamido, methoxycarbonyl, ethoxycarbonyl, benzoyl, methylcarbonyl, piperidinylcarbonyl, morpholinylcarbonyl, trifluoromethylcarbonyl, aminocarbonyl, methylaminocarbonyl, dimethylaminocarbonyl, (C₁-C₆)-alkoxy or (C₃-C₆)-cycloalkyl, or heteroaryl or heterocyclyl, each of which is substituted by p radicals from the group consisting of methyl, ethyl, methoxy, trifluoromethyl and halogen,
Y and Z are each independently O or S,
W¹ is (C₁-C₁₀)-alkylene, (C₂-C₆)-alkenylene or (C₂-C₆)-alkynylene,
W² is (C₁-C₁₀)-alkylene,
G is heteroaryl or heterocyclyl, each of which is substituted by s radicals from the group consisting of halogen, nitro, cyano, (C₁-C₆)-alkyl, halo- (C₁-C₆)-alkyl, (C₃-C₆)-cycloalkyl, S(O)ₙ-(C₁-C₆)-alkyl, (C₁-C₆)-alkoxy, halo-(C₁-C₆)-alkoxy and (C₁-C₆)-alkoxy-(C₁-C₄)-alkyl, where heterocyclyl bears n oxo groups,
R⁶ is halogen, cyano, hydroxyl, amino, nitro, -CHO,-C(=O)OH,
-C(=O)NH₂, -C(=S)NH₂, -C(=O)NHCN, -C(=O)NHOH, -SH,-SO₂NH₂, -SO₂NHCN,
-SO₂NHOH, -OCN, -SCN, -SF₅, (C₁-C₆)-alkyl, (C₂-C₆)-alkenyl, (C₂-C₆)-alkynyl,
halo-(C₁-C₆)-alkyl, halo-(C₂-C₆)-alkenyl, halo-(C₂-C₆)-alkynyl, (C₃-C₈) -cycloalkyl, halo-(C₃-C₈)-cycloalkyl, (C₁-C₆)-alkyl-(C₃-C₇)-cycloalkyl, (C₃-C₇)-cycloalkyl-(C₁-C₆)-alkyl, (C₃-C₈)-cycloalkenyl, halo-(C₃-C₈)-cycloalkenyl, (C₁-C₆)-alkoxy-(C₁-C₆)-alkyl, (C₃-C₇)-cycloalkoxy-(C₁-C₆)-alkyl, (C₁-C₆)-alkoxy-(C₁-C₆)-alkoxy-(C₁-C₆)-alkyl, (C₁-C₆)-alkylthio-(C₁-C₆)-alkyl, (C₁-C₈)-alkylsulfinyl-(C₁-C₆)-alkyl, (C₁-C₈)-alkoxyhalo-(C₁-C₆)-alkyl, cyano-(C₁-C₆)-alkyl, hydroxy-(C₁-C₆)-alkyl, (C₁-C₆)-alkoxy, halo-(C₁-C₆)-alkoxy, (C₃-C₈)-cycloalkoxy, halo-(C₃-C₈)-cycloalkoxy, (C₃-C₈)-cycloalkyl-(C₁-C₆)-alkoxy, (C₂-C₆)-alkenyloxy, halo-(C₂-C₆)-alkenyloxy, (C₁-C₆)-alkoxy-(C₁-C₆)-alkoxy, (C₂-C₆)-alkylcarbonyloxy, (C₁-C₆) -alkylthio, halo-(C₁-C₆)-alkylthio, (C₃-C₈)-cycloalkylthio, (C₁-C₆)-alkylsulfinyl, halo-(C₁-C₆)-alkylsulfinyl, (C₁-C₆)-alkylsulfonyl, halo-(C₁-C₆)-alkylsulfonyl, (C₃-C₈)-cycloalkylsulfonyl, (C₁-C₆)-alkylamino, di-(C₁-C₆)-alkylamino, halo-(C₁-C₆)-alkylamino, halodi-(C₁-C₈)-alkylamino or (C₃-C₈)-cycloalkylamino, or two vicinal R⁶ radicals form a (C₃-C₆)-alkylene group in which p carbon atoms are replaced by oxygen, sulfur or nitrogen, and in which one carbon atom bears t oxo groups,
n is 0, 1 or 2,
p is 0, 1, 2 or 3,
s is 0, 1, 2, 3, 4 or 5,
t is 0 or 1.

2. 1,2,4-Triazine-3,5-dione-6-carboxamides of the formula (I) or salts thereof according to Claim 1, in which
R¹ is (C₁-C₁₀)-alkyl, (C₂-C₁₀)-alkenyl, (C₂-C₁₀)-alkynyl, halo-(C₁-C₁₀)-alkyl, halo-(C₂-C₁₀)-alkenyl, halo-(C₂-C₁₀)-alkynyl, (C₃-C₁₀)-cycloalkyl, halo-(C₃-C₁₀)-cycloalkyl, (C₁-C₄)-alkyl-(C₃**-**C₇)-cycloalkyl, (C₃-C₇)-cycloalkyl-(C₁-C₆)-alkyl, (C₃-C₇)-cycloalkyl- (C₃-C₇)-cycloalkyl, halo-(C₃-C₇)-cycloalkyl- (C₁-C₆)-alkyl, (C₁-C₆) -alkyl- (C₃-C₇) -cycloalkyl- (C₁-C₆)-alkyl, (C₃-C₁₂)-cycloalkenyl, halo-(C₃**-**C₁₂)-cycloalkenyl, (C₁-C₆)-alkoxy-(C₁-C₆)-alkyl, (C₁-C₆)-alkoxy- (C₂-C₆)-alkenyl, (C₁-C₆)-alkyl-(C₃-C₇)-cycloalkyl, (C₁-C₆)-alkoxy- (C₃-C₇)-cycloalkyl, (C₃-C₇)-cycloalkoxy-(C₁-C₆)-alkyl, (C₃-C₇)-cycloalkoxy-(C₁-C₆)-alkoxy-(C₁-C₆)-alkyl, (C₁-C₆)-alkoxy-(C₁-C₆)-alkoxy-(C₁-C₆)-alkyl, (C₁-C₆)-alkylthio-(C₁-C₆)-alkyl, (C₁-C₆)-alkylsulfinyl-(C₁-C₆)-alkyl, (C₁-C₆)-alkylsulfonyl-(C₁-C₆)-alkyl, (C₁-C₆)-alkylamino-(C₁-C₆)-alkyl, di-(C₁-C₆)-alkylamino-(C₁-C₆)-alkyl, halo-(C₁-C₆)-alkylamino-(C₁-C₆)-alkyl, (C₃-C₇)-cycloalkylamino-(C₁-C₆)-alkyl, (C₁-C₆)-alkylcarbonyl, halo-(C₁-C₆)-alkylcarbonyl, (C₃-C₇)-cycloalkylcarbonyl, (C₁-C₆)-alkoxycarbonyl, (C₃-C₇)-cycloalkoxycarbonyl, (C₃-C₇)-cycloalkyl- (C₁-C₆)-alkoxycarbonyl, (C₁-C₆)-alkylaminocarbonyl, di-(C₁-C₆)-alkylaminocarbonyl, (C₃-C₇)-cycloalkylaminocarbonyl, cyano-(C₁-C₆)-alkyl, hydroxy-(C₁-C₆)-alkyl, (C₃-C₇)-cycloalkenyl-(C₁-C₆)-alkyl, halo-(C₁-C₆)-alkoxy-(C₁-C₆)-alkyl, (C₁-C₆)-alkoxyhalo-(C₁-C₆)-alkyl, halo-(C₁-C₆)-alkoxyhalo-(C₁-C₆)-alkyl, halo-(C₃-C₇)-cycloalkoxy-(C₁-C₆)-alkyl, (C₃-C₇)-cycloalkenyloxy-(C₁-C₆)-alkyl, halo-(C₃-C₇)-cycloalkenyloxy-(C₁-C₆)-alkyl, di-(C₁-C₆)-alkoxy-(C₁-C₆)-alkyl, (C₁-C₆)-alkoxy-(C₁-C₆)-alkylcarbonyl, (C₁-C₆)-alkoxycarbonyl-(C₁-C₆)-alkyl, halo-(C₁-C₆)-alkoxycarbonyl, (C₁-C₁₀)-alkoxy, halo-(C₁-C₁₀)-alkoxy, (C₃-C₁₂)-cycloalkoxy, halo-(C₃-C₇)-cycloalkoxy, (C₃-C₇)-cycloalkyl-(C₁-C₆)-alkoxy, (C₂-C₁₂)-alkenyloxy, halo-(C₂-C₁₀)-alkenyloxy, (C₂-C₁₀)-alkynyloxy, halo-(C₃-C₁₀)-alkynyloxy, (C₁-C₆)-alkoxy-(C₁-C₆)-alkoxy, (C₁-C₆)-alkylcarbonyloxy, halo-(C₂-C₁₂)-alkylcarbonyloxy, (C₃-C₇)-cycloalkylcarbonyloxy, (C₁-C₆)-alkylcarbonyl- (C₁-C₆)-alkoxy, (C₁-C₁₀)-alkylthio, halo-(C₁-C₁₀)-alkylthio, (C₃-C₁₂)-cycloalkylthio, (C₁-C₁₀)-alkylsulfinyl, halo-(C₁-C₁₀)-alkylsulfinyl, (C₁-C₁₀)-alkylsulfonyl, halo-(C₁-C₁₀)-alkylsulfonyl, (C₃-C₁₂)-cycloalkylsulfonyl, (C₁-C₆)-alkylcarbonylthio, (C₁-C₆)-alkyl(thiocarbonyl)thio, (C₃-C₁₂)-cycloalkylsulfinyl, (C₁-C₆)-alkylaminosulfonyl, di-(C₁-C₆)-alkylaminosulfonyl, (C₁-C₆)-alkylamino, di-(C₁-C₆)-alkylamino, halo-(C₁-C₆)-alkylamino, halodi-(C₁-C₆)-alkylamino, (C₃-C₁₂)-cycloalkylamino, (C₁-C₆)-alkylcarbonylamino, halo-(C₁-C₆)-alkylcarbonylamino, (C₁-C₁₀)-alkylsulfonylamino, halo-(C₁-C₁₀)-alkylsulfonylamino, (C₃-C₇)-cycloalkyl-(C₁-C₆)-alkylamino, cyano, hydroxyl, amino, C(=O)OH, C(=O)NHOH, SO₂NH₂, SO₂NHCN, SO₂NHOH, NHCHO, or
R¹ is phenyl, phenylsulfonyl, W¹-(phenyl), W¹-(O-phenyl), W¹-(S-phenyl), W¹-(SO₂-phenyl), W²-(SO₂CH₂-phenyl) or W²-(SCH₂-phenyl), where the phenyl rings of the seven aforementioned radicals each bear s R⁶ substituents, or
R¹ is G or W²G,
R² is hydrogen, (C₁-C₄)-alkyl, (C₁-C₄)-haloalkyl, (C₂-C₆)-alkenyl, (C₂-C₆-haloalkenyl, (C₂-C₆-alkynyl, (C₂-C₆)-haloalkynyl, (C₃-C₆-cycloalkyl, (C₃-C₆)-halocycloalkyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, where the phenyl or hetaryl rings of the four aforementioned radicals each bear s R⁶ substituents,
in which Q is a Q¹, Q², Q³ or Q⁴ radical, 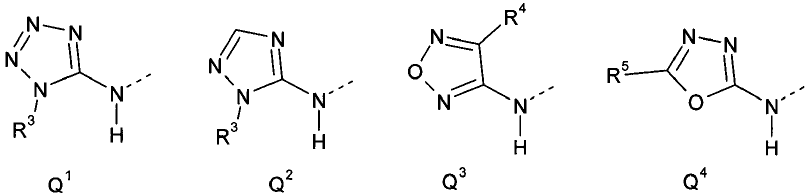 R³ is (C₁-C₈)-alkyl, (C₂-C₈)-alkenyl, (C₂-C₈)-alkynyl, where these radicals are each substituted by s radicals from the group consisting of halogen, cyano, nitro, S(O)ₙ-(C₁-C₆)-alkyl, (C₁-C₆)-alkoxy, halo-(C₁-C₆)-alkoxy, (C₃-C₆)-cycloalkyl, heteroaryl, heterocyclyl or phenyl, where the 4 latter radicals are each substituted by p radicals from the group consisting of methyl, ethyl, methoxy, trifluoromethyl, cyano and halogen, and where heterocyclyl bears n oxo groups, or
R³ is phenyl substituted in each case by p radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy, halo-(C₁-C₆)-alkoxy and (C₁-C₆)-alkoxy-(C₁-C₄)-alkyl,
R⁴ is (C₁-C₆)-alkyl, (C₃-C₇)-cycloalkyl, halo-(C₁-C₆)-alkyl, (C₁-C₆)-alkoxy, halo-(C₁-C₆)-alkoxy, cyano, nitro, methylsulfenyl, methylsulfinyl, methylsulfonyl, (C₁-C₄)-alkylcarbonylamino, benzoylamino, methoxycarbonyl, ethoxycarbonyl, benzoyl, phenoxy, methylcarbonyl, piperidinylcarbonyl, trifluoromethylcarbonyl, halogen, amino, aminocarbonyl, methylaminocarbonyl, dimethylaminocarbonyl, methoxymethyl, 1,2,4-triazol-1-yl, pyrazol-1-yl, 2-thiophenyl, 2-pyridinyl, 3-pyridinyl, 4-pyridinyl, 1,2,4-oxadiazol-3-yl, benzoxazol-2-yl, 1-ethylbenzimidazol-2-yl or piperidin-1-yl, or phenyl in each case substituted by p radicals from the group consisting of methyl, ethyl, methoxy, trifluoromethyl and halogen,
R⁵ is hydrogen, (C₁-C₆)-alkyl, (C₃-C₇)-cycloalkyl, halo- (C₁-C₆)-alkyl, (C₃-C₇)-cycloalkylmethyl, methoxycarbonylmethyl, ethoxycarbonylmethyl, acetylmethyl, methoxymethyl, methoxyethyl, benzyl, pyrazin-2-yl, furan-2-yl, tetrahydrofuran-2-yl, morpholine or dimethylamino, or phenyl substituted by p radicals from the group consisting of methyl, methoxy, trifluoromethyl and halogen,
R⁶ is halogen, cyano, hydroxyl, amino, nitro, -CHO,-C(=O)OH,
-C(=O)NH₂, -C(=S)NH₂, -C(=O)NHCN, -C(=O)NHOH, -SH, - SO₂NH₂, -SO₂NHCN,
-SO₂NHOH, -OCN, -SCN, -SF₅, (C₁-C₆)-alkyl, (C₂-C₆)-alkenyl, (C₂-C₆)-alkynyl,
halo-(C₁-C₆)-alkyl, halo-(C₂-C₆)-alkenyl, halo-(C₂-C₆)-alkynyl, (C₃-C₈)-cycloalkyl, halo-(C₃-C₈)-cycloalkyl, (C₁-C₆)-alkyl- (C₃-C₇)-cycloalkyl, (C₃-C₇)-cycloalkyl- (C₁-C₆)-alkyl, (C₃-C₈)-cycloalkenyl, halo-(C₃-C₈)-cycloalkenyl, (C₁-C₆)-alkoxy-(C₁-C₆)-alkyl, (C₃-C₇)-cycloalkoxy-(C₁-C₆)-alkyl, (C₁-C₆)-alkoxy-(C₁-C₆)-alkoxy-(C₁-C₆) -alkyl, (C₁-C₆)-alkylthio-(C₁-C₆)-alkyl, (C₁-C₈)-alkylsulfinyl-(C₁-C₆)-alkyl, (C₁-C₈)-alkoxyhalo-(C₁-C₆)-alkyl, cyano-(C₁-C₆)-alkyl, hydroxy-(C₁-C₆)-alkyl, (C₁-C₆-alkoxy, halo-(C₁-C₆)-alkoxy, (C₃-C₈)-cycloalkoxy, halo-(C₃-C₈)-cycloalkoxy, (C₃-C₈)-cycloalkyl-(C₁-C₆)-alkoxy, (C₂-C₆)-alkenyloxy, halo-(C₂-C₆) -alkenyloxy, (C₁-C₆)-alkoxy-(C₁-C₆)-alkoxy, (C₂-C₆) -alkylcarbonyloxy, (C₁-C₆-alkylthio, halo-(C₁-C₆)-alkylthio, (C₃-C₈)-cycloalkylthio, (C₁-C₆)-alkylsulfinyl, halo-(C₁-C₆)-alkylsulfinyl, (C₁-C₆)-alkylsulfonyl, halo-(C₁-C₆)-alkylsulfonyl, (C₃-C₈)-cycloalkylsulfonyl, (C₁-C₆)-alkylamino, di-(C₁-C₆)-alkylamino, halo-(C₁-C₆)-alkylamino, halodi-(C₁-C₈)-alkylamino or (C₃-C₈)-cycloalkylamino, or two vicinal R⁶ radicals form a (C₃-C₆)-alkylene group in which p carbon atoms are replaced by oxygen, sulfur or nitrogen, and in which one carbon atom bears t oxo groups,
Y and Z are each independently 0 or S,
W¹ is (C₁-C₁₀)-alkylene, (C₂-C₆)-alkenylene or (C₂-C₆)-alkynylene,
W² is (C₁-C₁₀)-alkylene,
G is heteroaryl or heterocyclyl, each of which is substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy, halo-(C₁-C₆)-alkoxy and (C₁-C₆)-alkoxy-(C₁-C₄)-alkyl, where heterocyclyl bears n oxo groups,
n is 0, 1 or 2,
p is 0, 1, 2 or 3,
s is 0, 1, 2, 3, 4 or 5,
t is 0 or 1.

3. 1,2,4-Triazine-3,5-dione-6-carboxamides of the formula (I) or salts thereof according to Claim 1 or 2, in which
R¹ is (C₁-C₁₀)-alkyl, (C₂-C₁₀)-alkenyl, (C₂-C₁₀)-alkynyl, halo-(C₁-C₁₀)-alkyl, halo-(C₂-C₁₀)-alkenyl, halo-(C₂-C₁₀)-alkynyl, (C₃-C₁₀)-cycloalkyl, halo-(C₃-C₁₀)-cycloalkyl, (C₁-C₄)-alkyl-(C₃-C₇)-cycloalkyl, (C₃-C₇)-cycloalkyl-(C₁-C₆)-alkyl, (C₃-C₇)-cycloalkyl-(C₃-C₇)-cycloalkyl, halo- (C₃-C₇)-cycloalkyl- (C₁-C₆)-alkyl, (C₁-C₆)-alkyl-(C₃-C₇) -cycloalkyl-(C₁-C₆)-alkyl, (C₃-C₁₂)-cycloalkenyl, halo- (C₃-C₁₂) -cycloalkenyl, (C₁-C₆)-alkoxy-(C₁-C₆)-alkyl, (C₁-C₆)-alkoxy-(C₂-C₆)-alkenyl, (C₁-C₆)-alkyl-(C₃-C₇)-cycloalkyl, (C₁-C₆)-alkoxy- (C₃-C₇)-cycloalkyl, (C₃**-**C₇)-cycloalkoxy-(C₁-C₆)-alkyl, (C₃-C₇)-cycloalkoxy-(C₁-C₆)-alkoxy-(C₁-C₆)-alkyl, (C₁-C₆)-alkoxy-(C₁-C₆)-alkoxy-(C₁-C₆)-alkyl, (C₁-C₆)-alkylthio-(C₁-C₆)-alkyl, (C₁-C₆)-alkylsulfinyl-(C₁-C₆)-alkyl, (C₁-C₆)-alkylsulfonyl-(C₁-C₆)-alkyl, (C₁-C₆)-alkylamino-(C₁-C₆)-alkyl, di-(C₁-C₆)-alkylamino-(C₁-C₆)-alkyl, halo-(C₁-C₆)-alkylamino-(C₁-C₆)-alkyl, (C₃-C₇)-cycloalkylamino- (C₁-C₆)-alkyl, (C₁-C₆)-alkylcarbonyl, halo-(C₁-C₆)-alkylcarbonyl, (C₃-C₇)-cycloalkylcarbonyl, (C₁-C₆)-alkoxycarbonyl, (C₃-C₇)-cycloalkoxycarbonyl, (C₃-C₇)-cycloalkyl- (C₁-C₆) -alkoxycarbonyl, (C₁-C₆)-alkylaminocarbonyl, di-(C₁-C₆)-alkylaminocarbonyl, (C₃-C₇)-cycloalkylaminocarbonyl, cyano-(C₁-C₆)-alkyl, hydroxy-(C₁-C₆)-alkyl, (C₃-C₇)-cycloalkenyl-(C₁-C₆)-alkyl, halo-(C₁-C₆)-alkoxy-(C₁-C₆)-alkyl, (C₁-C₆)-alkoxyhalo-(C₁-C₆)-alkyl, halo-(C₁-C₆)-alkoxyhalo-(C₁-C₆)-alkyl, halo-(C₃-C₇)-cycloalkoxy-(C₁-C₆)-alkyl, (C₃-C₇)-cycloalkenyloxy-(C₁-C₆)-alkyl, halo-(C₃-C₇)-cycloalkenyloxy-(C₁-C₆)-alkyl, di-(C₁-C₆)-alkoxy-(C₁-C₆)-alkyl, (C₁-C₆)-alkoxy-(C₁-C₆)-alkylcarbonyl, (C₁-C₆)-alkoxycarbonyl-(C₁-C₆)-alkyl, halo-(C₁-C₆)-alkoxycarbonyl, (C₁-C₁₀)-alkoxy, halo-(C₁-C₁₀)-alkoxy, (C₃-C₁₂)-cycloalkoxy, halo-(C₃-C₇)-cycloalkoxy, (C₃-C₇)-cycloalkyl-(C₁-C₆)-alkoxy, (C₂-C₁₂)-alkenyloxy, halo-(C₂-C₁₀)-alkenyloxy, (C₂-C₁₀)-alkynyloxy, halo-(C₃-C₁₀)-alkynyloxy, (C₁-C₆)-alkoxy-(C₁-C₆)-alkoxy, (C₁-C₆)-alkylcarbonyloxy, halo- (C₂-C₁₂)-alkylcarbonyloxy, (C₃-C₇)-cycloalkylcarbonyloxy, (C₁-C₆)-alkylcarbonyl- (C₁-C₆)-alkoxy, (C₁-C₁₀)-alkylthio, halo-(C₁-C₁₀)-alkylthio, (C₃-C₁₂)-cycloalkylthio, (C₁-C₁₀)-alkylsulfinyl, halo-(C₁-C₁₀)-alkylsulfinyl, (C₁-C₁₀)-alkylsulfonyl, halo-(C₁-C₁₀)-alkylsulfonyl, (C₃-C₁₂)-cycloalkylsulfonyl, (C₁-C₆)-alkylcarbonylthio, (C₁-C₆)-alkyl(thiocarbonyl)thio, (C₃-C₁₂)-cycloalkylsulfinyl, (C₁-C₆)-alkylaminosulfonyl, di-(C₁-C₆)-alkylaminosulfonyl, (C₁-C₆)-alkylamino, di-(C₁-C₆)-alkylamino, halo-(C₁-C₆)-alkylamino, halodi-(C₁-C₆)-alkylamino, (C₃-C₁₂)-cycloalkylamino, (C₁-C₆)-alkylcarbonylamino, halo-(C₁-C₆)-alkylcarbonylamino, (C₁-C₁₀)-alkylsulfonylamino, halo-(C₁-C₁₀)-alkylsulfonylamino, (C₃-C₇)-cycloalkyl-(C₁-C₆)-alkylamino, cyano, hydroxyl, amino, C(=O)OH, C(=O)NHOH, SO₂NH₂, SO₂NHCN, SO₂NHOH, NHCHO, or
R¹ is phenyl, W¹-(phenyl), where the phenyl rings each bear s R⁶ substituents, or
R¹ is G,
R² is (C₁-C₄)-alkyl, (C₁-C₄)-haloalkyl, phenyl, heteroaryl, where the phenyl or heteroaryl rings in the two aforementioned radicals each bear s R⁶ substituents,
Q is a Q¹, Q², Q³ or Q⁴ radical, 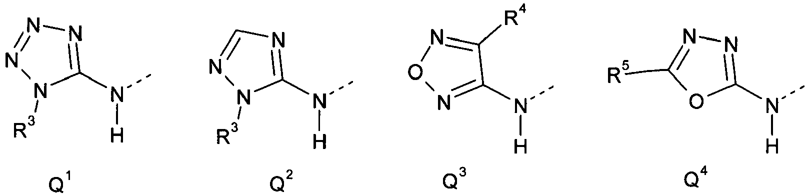 R³ is (C₁-C₈)-alkyl, (C₂-C₈)-alkenyl or (C₂-C₈)-alkynyl, each of which is substituted by s radicals from the group consisting of halogen, cyano, nitro, S(O)ₙ-(C₁-C₆)-alkyl, (C₁-C₆)-alkoxy and halo-(C₁-C₆)-alkoxy,
R⁴ is (C₁-C₆)-alkyl, (C₃-C₇-cycloalkyl, halo-(C₁-C₆)-alkyl, (C₁-C₆)-alkoxy, halo-(C₁-C₆)-alkoxy, cyano, nitro, methylsulfenyl, methylsulfinyl, methylsulfonyl, (C₁-C₄)-alkylcarbonylamino, benzoylamino, methoxycarbonyl, ethoxycarbonyl, benzoyl, phenoxy, methylcarbonyl, piperidinylcarbonyl, trifluoromethylcarbonyl, halogen, amino, aminocarbonyl, methylaminocarbonyl, dimethylaminocarbonyl, methoxymethyl, 1,2,4-triazol-1H, 1-pyrazol-1H, 2-thiophenyl, 2-pyridinyl, 3-pyridinyl, 4-pyridinyl, 1,2,4-oxadiazol-3-yl, benzoxazol-2-yl, 1-ethylbenzimidazol-2-yl or piperidin-1-yl, or phenyl in each case substituted by s radicals from the group consisting of methyl, ethyl, methoxy, trifluoromethyl and halogen,
R⁵ is hydrogen, (C₁-C₆)-alkyl, (C₃-C₇)-cycloalkyl, halo- (C₁-C₆)-alkyl, (C₃-C₇)-cycloalkylmethyl, methoxycarbonylmethyl, ethoxycarbonylmethyl, acetylmethyl, methoxymethyl, methoxyethyl, benzyl, pyrazin-2-yl, furan-2-yl, tetrahydrofuran-2-yl, morpholine or dimethylamino, or phenyl substituted by p radicals from the group consisting of methyl, methoxy, trifluoromethyl and halogen,
R⁶ is halogen, cyano, nitro, (C₁-C₆)-alkyl, (C₂-C₆) alkenyl, (C₂-C₆)-alkynyl,
halo-(C₁-C₆)-alkyl, (C₃-C₆)-cycloalkyl, (C₁-C₆)-alkoxy, halo-(C₁-C₆)-alkoxy, (C₁-C₆)-alkylthio, (C₁-C₆)-alkylsulfinyl, (C₁-C₆)-alkylsulfonyl, phenyl, methylenedioxo,
Y and Z are each independently 0 or S,
W¹ is (C₁-C₁₀)-alkylene,
G is heteroaryl or heterocyclyl, each of which is substituted by s radicals from the group consisting of halogen, nitro, cyano, (C₁-C₆)-alkyl, halo-(C₁-C₃)-alkyl, cyclopropyl, S(O)ₙ-(C₁-C₃)-alkyl, (C₁-C₄)-alkoxy, halo-(C₁-C₄)-alkoxy and (C₁-C₄)-alkoxy-(C₁-C₄)-alkyl, where heterocyclyl bears n oxo groups,
n is 0, 1 or 2,
p is 0, 1, 2 or 3,
s is 0, 1, 2, 3, 4 or 5.

4. Herbicidal compositions comprising a herbicidally effective amount of at least one compound of the formula (I) according to any of Claims 1 to 3.

5. Herbicidal compositions according to Claim 4 in a mixture with formulating auxiliaries.

6. Herbicidal compositions according to Claim 4 or 5, comprising at least one further pesticidally active substance from the group consisting of insecticides, acaricides, herbicides, fungicides, safeners, and growth regulators.

7. Herbicidal compositions according to Claim 6, comprising a safener.

8. Herbicidal compositions according to Claim 7, comprising cyprosulfamide, cloquintocet-mexyl, mefenpyr-diethyl or isoxadifen-ethyl.

9. Herbicidal compositions according to any of Claims 4 to 8, comprising a further herbicide.

10. Method for controlling unwanted plants, **characterized in that** an effective amount of at least one compound of the formula (I) according to any of Claims 1 to 3 or of a herbicidal composition according to any of Claims 4 to 8 is applied to the plants or to the site of the unwanted plant growth.

11. Use of compounds of the formula (I) according to any of Claims 1 to 3 or of herbicidal compositions according to any of Claims 4 to 8 for controlling unwanted plants.

12. Use according to Claim 11, **characterized in that** the compounds of the formula (I) are used for controlling unwanted plants in crops of useful plants.

13. Use according to Claim 12, **characterized in that** the useful plants are transgenic useful plants.

## Revendications

1. 1,2,4-Triazine-3,5-dione-6-carboxamides de formule (I) ou leurs sels 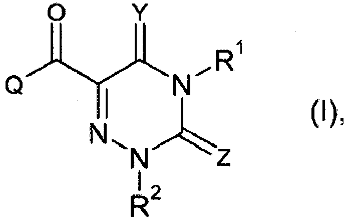 dans lesquels les substituants ont les significations suivantes :
R¹ signifie alkyle en (C₁-C₁₀), alcényle en (C₂-C₁₀), alcynyle en (C₂-C₁₀), halogéno-alkyle en (C₁-C₁₀), halogéno-alcényle en (C₂-C₁₀), halogéno-alcynyle en (C₂-C₁₀), cycloalkyle en (C₃-C₁₀), halogéno-cycloalkyle en (C₃-C₁₀), alkyle en (C₁-C₄)-cycloalkyle en (C₃-C₇), cycloalkyle en (C₃-C₇)-alkyle en (C₁-C₆), cycloalkyle en (C₃-C₇)-cycloalkyle en (C₃-C₇), halogéno-cycloalkyle en (C₃-C₇)-alkyle en (C₁-C₆), alkyle en (C₁-C₆)-cycloalkyle en (C₃-C₇)-alkyle en (C₁-C₆), cycloalcényle en (C₃-C₁₂), halogéno-cycloalcényle en (C₃-C₁₂), alcoxy en (C₁-C₆)-alkyle en (C₁-C₆), alcoxy en (C₁-C₆)-alcényle en (C₂-C₆), alkyle en (C₁-C₆)-cycloalkyle en (C₃-C₇), alcoxy en (C₁-C₆)-cycloalkyle en (C₃-C₇), cycloalcoxy en (C₃-C₇)-alkyle en (C₁-C₆), cycloalcoxy en (C₃-C₇)-alcoxy en (C₁-C₆)-alkyle en (C₁-C₆), alcoxy en (C₁-C₆)-alcoxy en (C₁-C₆)-alkyle en (C₁-C₆), alkylthio en (C₁-C₆)-alkyle en (C₁-C₆), alkylsulfinyle en (C₁-C₆)-alkyle en (C₁-C₆), alkylsulfonyle en (C₁-C₆)-alkyle en (C₁-C₆), alkylamino en (C₁-C₆) -alkyle en (C₁-C₆), di-alkylamino en (C₁-C₆)-alkyle en (C₁-C₆), halogéno-alkylamino en (C₁-C₆)-alkyle en (C₁-C₆) , cycloalkylamino en (C₃-C₇), alkyle en (C₁-C₆), alkyle en (C₁-C₆)-carbonyle, halogéno-alkyle en (C₁-C₆)-carbonyle, cycloalkyle en (C₃₋-C₇-carbonyle, alcoxy en (C₁-C₆)-carbonyle, cycloalcoxy en (C₃-C₇)-carbonyle, cycloalkyle en (C₃-C₇)-alcoxy en (C₁-C₆)-carbonyle, alkylamino en (C₁-C₆)-carbonyle, di-alkylamino en (C₁-C₆)-carbonyle, cycloalkylamino en (C₃-C₇)-carbonyle, cyano-alkyle en (C₁-C₆), hydroxy-alkyle en (C₁-C₆), cycloalcényle en (C₃-C₇)-alkyle en (C₁-C₆), halogéno-alcoxy en (C₁-C₆)-alkyle en (C₁-C₆), alcoxy en (C₁-C₆)-halogéno-alkyle en (C₁-C₆), halogéno-alcoxy en (C₁-C₆)-halogéno-alkyle en (C₁-C₆), halogéno-cycloalcoxy en (C₃-C₇)-alkyle en (C₁-C₆), cycloalcényloxy en (C₃-C₇)-alkyle en (C₁-C₆), halogéno-cycloalcényloxy en (C₃-C₇)-alkyle en (C₁-C₆), di-alcoxy en (C₁-C₆)-alkyle en (C₁-C₆), alcoxy en (C₁-C₆)-alkyle en (C₁-C₆)-carbonyle, alcoxy en (C₁-C₆)-carbonyl-alkyle en (C₁-C₆), halogéno-alcoxy en (C₁-C₆)-carbonyle, alcoxy en (C₁-C₁₀), halogéno-alcoxy en (C₁-C₁₀), cycloalcoxy en (C₃-C₁₂), halogéno-cycloalcoxy en (C₃-C₇), cycloalkyle en (C₃-C₇)-alcoxy en (C₁-C₆), alcényloxy en (C₂-C₁₂), halogéno-alcényloxy en (C₂-C₁₀), alcynyloxy en (C₂-C₁₀), halogéno-alcynyloxy en (C₃-C₁₀), alcoxy en (C₁-C₆)-alcoxy en (C₁-C₆), alkyle en (C₁-C₆)-carbonyloxy, halogéno-alkyle en (C₂-C₁₂)-carbonyloxy, cycloalkyle en (C₃-C₇)-carbonyloxy, alkyle en (C₁-C₆)-carbonyl-alcoxy en (C₁-C₆), alkylthio en (C₁-C₁₀), halogéno-alkylthio en (C₁-C₁₀), cycloalkylthio en (C₃-C₁₂), alkylsulfinyle en (C₁-C₁₀), halogéno-alkylsulfinyle en (C₁-C₁₀), alkylsulfonyle en (C₁-C₁₀), halogéno-alkylsulfonyle en (C₁-C₁₀), cycloalkylsulfonyle en (C₃-C₁₂), alkyle en (C₁-C₆)-carbonylthio, alkyle en (C₁-C₆)-(thiocarbonyl)thio, cycloalkylsulfinyle en (C₃-C₁₂), alkylaminosulfonyle en (C₁-C₆), di-alkylamino en (C₁-C₆)-sulfonyle, alkylamino en (C₁-C₆), di-alkylamino en (C₁-C₆), halogéno-alkylamino en (C₁-C₆), halogéno-di-alkylamino en (C₁-C₆), cycloalkylamino en (C₃-C₁₂), alkyle en (C₁-C₆)-carbonylamino, halogéno-alkyle en (C₁-C₆)-carbonylamino, alkylsulfonylamino en (C₁-C₁₀), halogéno-alkylsulfonyamino en (C₁-C₁₀), cycloalkyle en (C₃-C₇)-alkyle en (C₁-C₆)-amino, cyano, hydroxy, amino, C(=O)OH, C(=O)NHOH, SO₂NH₂, SO₂NHCN, SO₂NHOH, NHCHO ou
R¹ signifie phényle, phénylsulfonyle, W¹-(phényle), W¹-(O-phényle), W¹-(S-phényle), W¹-(SO₂-phényle), W²-(SO₂CH₂-phényle) ou W²-(SCH₂-phényle), les cycles phényle des sept radicaux susmentionnés portant chacun s substituants R⁶, ou
R¹ signifie G, W²G ou W²OG,
R² signifie hydrogène, alkyle en (C₁-C₄), haloalkyle en (C₁-C₄), alcényle en (C₂-C₆), halogénoalcényle en (C₂-C₆), alcynyle en (C₂-C₆), halogénoalcynyle en (C₂-C₆), cycloalkyle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, les cycles phényle ou hétéroaryle des quatre radicaux susmentionnés portant à chaque fois s substituants R⁶,
Q signifie un radical Q¹, Q², Q³ ou Q⁴ 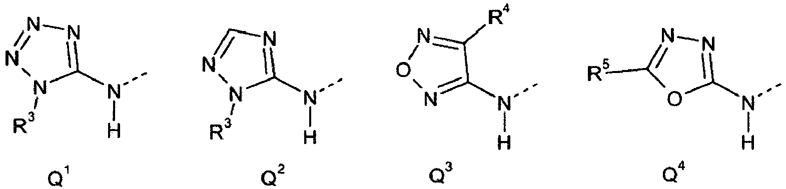
R³ signifie alkyle en (C₁-C₈), alcényle en (C₂-C₈), alcynyle en (C₂-C₈), ces radicaux étant chacun substitués par s radicaux du groupe constitué par halogène, cyano, hydroxy, nitro, SiR¹⁰₃, PO(OR¹⁰)₂, S(O)ₙ-alkyle en (C₁-C₆), alcoxy en (C₁-C₆), halogéno-alcoxy en (C₁-C₆), COR^{3a}, COOR^{3a}, OCOR^{3a}, NR^{3a}COR^{3a}, NR^{3a}SO₂R^{3b}, cycloalkyle en (C₃-C₆), hétéroaryle, hétérocyclyle ou phényle, les 4 derniers radicaux cités étant chacun substitués par p radicaux du groupe constitué par méthyle, éthyle, méthoxy, trifluorométhyle, cyano et halogène, et hétérocyclyle portant n groupes oxo, ou
R³ signifie phényle, qui est substitué par p radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆), alcoxy en (C₁-C₆)-alkyle en (C₁-C₄),
R^{3a} signifie hydrogène, alkyle en (C₁-C₆), alcényle en (C₂-C₆), alcynyle en (C₂-C₆), cycloalkyle en (C₃-C₆) ou cycloalkyle en (C₃-C₆)-alkyle en (C₁-C₆) ou phényle,
R^{3b} signifie alkyle en (C₁-C₆), alcényle en (C₂-C₆) ou alcynyle en (C₂-C₆), cycloalkyle en (C₃-C₆) ou cycloalkyle en (C₃-C₆)-alkyle en (C₁-C₆) ou phényle,
R⁴ signifie hydrogène, alkyle en (C₁-C₆), cycloalkyle en (C₃-C₇), halogéno-alkyle en (C₁-C₆), alcoxy en (C₁-C₆), halogéno-alcoxy en (C₁-C₆), alcényle en (C₂-C₆), alcényloxy en (C₂-C₆), halogénoalcényle en (C₂-C₆), alcynyle en (C₂-C₆), alcynyloxy en (C₂-C₆), halogénoalcynyle en (C₂-C₆), alcoxy en (C₁-C₆) -alkyle en (C₁-C₆), cyano, nitro, méthylsulfényle, méthylsulfinyle, méthylsulfonyle, acétylamino, benzoylamino, méthoxycarbonyle, éthoxycarbonyle, méthoxycarbonylméthyle, éthoxycarbonylméthyle, benzoyle, méthylcarbonyle, pipéridinylcarbonyle, trifluorométhylcarbonyle, halogène, amino, aminocarbonyle, méthylaminocarbonyle, diméthylaminocarbonyle, méthoxyméthyle, ou hétéroaryle, hétérocyclyle ou phényle, chacun substitué par p radicaux du groupe constitué par méthyle, éthyle, méthoxy, trifluorométhyle et halogène,
R⁵ signifie hydrogène, alkyle en (C₁-C₆), alcoxy en (C₁-C₆)-alkyle en (C₁-C₆), CH₂R^{5a}, cycloalkyle en (C₃-C₇), halogéno-alkyle en (C₁-C₆), alcényle en (C₂-C₆), halogéno-alcényle en (C₂-C₆), alcynyle en (C₂-C₆), halogéno-alcynyle en (C₂-C₆), OR⁶, NHR⁶, méthoxycarbonyle, éthoxycarbonyle, méthoxycarbonylméthyle, éthoxycarbonylméthyle, méthylcarbonyle, trifluorométhylcarbonyle, diméthylamino, acétylamino, méthylsulfényle, méthylsulfinyle, méthylsulfonyle, ou hétéroaryle, hétérocyclyle, benzyle ou phényle, chacun substitué par p radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆), alcoxy en (C₁-C₆)-alkyle en (C₁-C₄),
R^{5a} signifie acétoxy, acétamido, N-méthylacétamido, benzoyloxy, benzamido, N-méthylbenzamido, méthoxycarbonyle, éthoxycarbonyle, benzoyle, méthylcarbonyle, pipéridinylcarbonyle, morpholinylcarbonyle, trifluorométhylcarbonyle, aminocarbonyle, méthylaminocarbonyle, diméthylaminocarbonyle, alcoxy en (C₁-C₆), cycloalkyle en (C₃-C₆), ou hétéroaryle ou hétérocyclyle, chacun substitué par p radicaux du groupe constitué par méthyle, éthyle, méthoxy, trifluorométhyle et halogène,
Y et Z signifient chacun indépendamment l'un de l'autre 0 ou S,
W¹ signifie alkylène en (C₁-C₁₀), alcénylène en (C₂-C₆) ou alcynylène en (C₂-C₆),
W² signifie alkylène en (C₁-C₁₀),
G signifie hétéroaryle ou hétérocyclyle, chacun substitué par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆) et alcoxy en (C₁-C₆)-alkyle en (C₁-C₄), hétérocyclyle portant n groupes oxo,
R⁶ signifie halogène, cyano, hydroxy, amino, nitro,-CHO, -C(=O)OH, -C(=O)NH₂, -C(=S)NH₂, -C(=O)NHCN,-C(=O)NHOH, -SH, -SO₂NH₂, -SO₂NHCN, -SO₂NHOH, -OCN, -SCN, -SF₅, alkyle en (C₁-C₆), alcényle en (C₂-C₆), alcynyle en (C₂-C₆), halogéno-alkyle en (C₁-C₆), halogéno-alcényle en (C₂-C₆), halogéno-alcynyle en (C₂-C₆), cycloalkyle en (C₃-C₈), halogéno-cycloalkyle en (C₃-C₈), alkyle en (C₁-C₆)-cycloalkyle en (C₃-C₇), cycloalkyle en (C₃-C₇)-alkyle en (C₁-C₆), cycloalcényle en (C₃-C₈), halogéno-cycloalcényle en (C₃-C₈), alcoxy en (C₁-C₆)-alkyle en (C₁-C₆), cycloalcoxy en (C₃-C₇)-alkyle en (C₁-C₆), alcoxy en (C₁-C₆)-alcoxy en (C₁-C₆)-alkyle en (C₁-C₆), alkylthio en (C₁-C₆)-alkyle en (C₁-C₆), alkylsulfinyle en (C₁-C₈)-alkyle en (C₁-C₆), alcoxy en (C₁-C₈)-halogéno-alkyle en (C₁-C₆), cyano-alkyle en (C₁-C₆), hydroxy-alkyle en (C₁-C₆), alcoxy en (C₁-C₆), halogéno-alcoxy en (C₁-C₆), cycloalcoxy en (C₃-C₈), halogéno-cycloalcoxy en (C₃-C₈), cycloalkyle en (C₃-C₈)-alcoxy en (C₁-C₆), alcényloxy en (C₂-C₆), halogéno-alcényloxy en (C₂-C₆), alcoxy en (C₁-C₆)-alcoxy en (C₁-C₆), alkylcarbonyloxy en (C₂-C₆), alkylthio en (C₁-C₆), halogéno-alkylthio en (C₁-C₆), cycloalkylthio en (C₃-C₈), alkylsulfinyle en (C₁-C₆), halogéno-alkylsulfinyle en (C₁-C₆), alkylsulfonyle en (C₁-C₆), halogéno-alkylsulfonyle en (C₁-C₆), cycloalkylsulfonyle en (C₃-C₈), alkylamino en (C₁-C₆), di-alkylamino en (C₁-C₆), halogéno-alkylamino en (C₁-C₆), halogéno-di-alkylamino en (C₁-C₈) ou cycloalkylamino en (C₃-C₈), ou deux radicaux R⁶ vicinaux forment un groupe alkylène en (C₃-C₆), dans lequel p atomes de carbone sont remplacés par oxygène, soufre ou azote, et dans lequel un atome de carbone porte t groupes oxo,
n signifie 0, 1 ou 2,
p signifie 0, 1, 2 ou 3,
s signifie 0, 1, 2, 3, 4 ou 5,
t signifie 0 ou 1.

2. 1,2,4-Triazine-3,5-dione-6-carboxamides de formule (I) ou leurs sels selon la revendication 1, dans lesquels
R¹ signifie alkyle en (C₁-C₁₀), alcényle en (C₂-C₁₀), alcynyle en (C₂-C₁₀), halogéno-alkyle en (C₁-C₁₀), halogéno-alcényle en (C₂-C₁₀), halogéno-alcynyle en (C₂-C₁₀), cycloalkyle en (C₃-C₁₀), halogéno-cycloalkyle en (C₃-C₁₀), alkyle en (C₁-C₄)-cycloalkyle en (C₃-C₇), cycloalkyle en (C₃-C₇)-alkyle en (C₁-C₆), cycloalkyle en (C₃-C₇)-cycloalkyle en (C₃-C₇), halogéno-cycloalkyle en (C₃-C₇)-alkyle en (C₁-C₆), alkyle en (C₁-C₆)-cycloalkyle en (C₃-C₇)-alkyle en (C₁-C₆), cycloalcényle en (C₃-C₁₂), halogéno-cycloalcényle en (C₃-C₁₂), alcoxy en (C₁-C₆)-alkyle en (C₁-C₆), alcoxy en (C₁-C₆)-alcényle en (C₂-C₆), alkyle en (C₁-C₆)-cycloalkyle en (C₃-C₇), alcoxy en (C₁-C₆)-cycloalkyle en (C₃-C₇), cycloalcoxy en (C₃-C₇)-alkyle en (C₁-C₆), cycloalcoxy en (C₃-C₇)-alcoxy en (C₁-C₆)-alkyle en (C₁-C₆), alcoxy en (C₁-C₆)-alcoxy en (C₁-C₆)-alkyle en (C₁-C₆), alkylthio en (C₁-C₆)-alkyle en (C₁-C₆), alkylsulfinyle en (C₁-C₆)-alkyle en (C₁-C₆), alkylsulfonyle en (C₁-C₆)-alkyle en (C₁-C₆), alkylamino en (C₁-C₆)-alkyle en (C₁-C₆), di-alkylamino en (C₁-C₆)-alkyle en (C₁-C₆), halogéno-alkylamino en (C₁-C₆)-alkyle en (C₁-C₆), cycloalkylamino en (C₃-C₇)-alkyle en (C₁-C₆), alkyle en (C₁-C₆)-carbonyle, halogéno-alkyle en (C₁-C₆)-carbonyle, cycloalkyle en (C₃₋-C₇)-carbonyle, alcoxy en (C₁-C₆)-carbonyle, cycloalcoxy en (C₃-C₇)-carbonyle, cycloalkyle en (C₃-C₇)-alcoxy en (C₁-C₆)-carbonyle, alkylamino en (C₁-C₆)-carbonyle, di-alkylamino en (C₁-C₆)-carbonyle, cycloalkylamino en (C₃-C₇)-carbonyle, cyano-alkyle en (C₁-C₆), hydroxy-alkyle en (C₁-C₆), cycloalcényle en (C₃-C₇)-alkyle en (C₁-C₆), halogéno-alcoxy en (C₁-C₆)-alkyle en (C₁-C₆), alcoxy en (C₁-C₆)-halogéno-alkyle en (C₁-C₆), halogéno-alcoxy en (C₁-C₆)-halogéno-alkyle en (C₁-C₆), halogéno-cycloalcoxy en (C₃-C₇)-alkyle en (C₁-C₆), cycloalcényloxy en (C₃-C₇)-alkyle en (C₁-C₆), halogéno-cycloalcényloxy en (C₃-C₇)-alkyle en (C₁-C₆), di-alcoxy en (C₁-C₆)-alkyle en (C₁-C₆), alcoxy en (C₁-C₆)-alkyle en (C₁-C₆)-carbonyle, alcoxy en (C₁-C₆)-carbonyl-alkyle en (C₁-C₆), halogéno-alcoxy en (C₁-C₆)-carbonyle, alcoxy en (C₁-C₁₀), halogéno-alcoxy en (C₁-C₁₀), cycloalcoxy en (C₃-C₁₂), halogéno-cycloalcoxy en (C₃-C₇), cycloalkyle en (C₃-C₇)-alcoxy en (C₁-C₆), alcényloxy en (C₂-C₁₂), halogéno-alcényloxy en (C₂-C₁₀), alcynyloxy en (C₂-C₁₀), halogéno-alcynyloxy en (C₃-C₁₀), alcoxy en (C₁-C₆)-alcoxy en (C₁-C₆), alkyle en (C₁-C₆)-carbonyloxy, halogéno-alkyle en (C₂-C₁₂)-carbonyloxy, cycloalkyle en (C₃-C₇)-carbonyloxy, alkyle en (C₁-C₆)-carbonyl-alcoxy en (C₁-C₆), alkylthio en (C₁-C₁₀), halogéno-alkylthio en (C₁-C₁₀), cycloalkylthio en (C₃-C₁₂), alkylsulfinyle en (C₁-C₁₀), halogéno-alkylsulfinyle en (C₁-C₁₀), alkylsulfonyle en (C₁-C₁₀), halogéno-alkylsulfonyle en (C₁-C₁₀), cycloalkylsulfonyle en (C₃-C₁₂), alkyle en (C₁-C₆)-carbonylthio, alkyle en (C₁-C₆)-(thiocarbonyl)thio, cycloalkylsulfinyle en (C₃-C₁₂), alkylaminosulfonyle en (C₁-C₆), di-alkylamino en (C₁-C₆)-sulfonyle, alkylamino en (C₁-C₆), di-alkylamino en (C₁-C₆), halogéno-alkylamino en (C₁-C₆), halogéno-di-alkylamino en (C₁-C₆), cycloalkylamino en (C₃-C₁₂), alkyle en (C₁-C₆)-carbonylamino, halogéno-alkyle en (C₁-C₆)-carbonylamino, alkylsulfonylamino en (C₁-C₁₀), halogéno-alkylsulfonyamino en (C₁-C₁₀), cycloalkyle en (C₃-C₇)-alkyle en (C₁-C₆)-amino, cyano, hydroxy, amino, C(=O)OH, C(=O)NHOH, SO₂NH₂, SO₂NHCN, SO₂NHOH, NHCHO ou
R¹ signifie phényle, phénylsulfonyle, W¹-(phényle), W¹-(O-phényle), W¹-(S-phényle), W¹-(SO₂-phényle), W²-(SO₂CH₂-phényle) ou W²-(SCH₂-phényle), les cycles phényle des sept radicaux susmentionnés portant chacun s substituants R⁶, ou
R¹ signifie G ou W²G,
R² signifie hydrogène, alkyle en (C₁-C₄), haloalkyle en (C₁-C₄), alcényle en (C₂-C₆), halogénoalcényle en (C₂-C₆), alcynyle en (C₂-C₆), halogénoalcynyle en (C₂-C₆), cycloalkyle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, les cycles phényle ou hétaryle des quatre radicaux susmentionnés portant à chaque fois s substituants R⁶,
Q signifie un radical Q¹, Q², Q³ ou Q⁴ 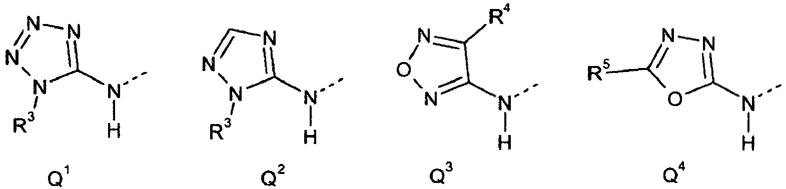 R³ signifie alkyle en (C₁-C₈), alcényle en (C₂-C₈), alcynyle en (C₂-C₈), ces radicaux étant chacun substitués par s radicaux du groupe constitué par halogène, cyano, nitro, S(O)ₙ-alkyle en (C₁-C₆), alcoxy en (C₁-C₆), halogéno-alcoxy en (C₁-C₆), cycloalkyle en (C₃-C₆), hétéroaryle, hétérocyclyle ou phényle, les 4 derniers radicaux cités étant chacun substitués par p radicaux du groupe constitué par méthyle, éthyle, méthoxy, trifluorométhyle, cyano et halogène, et hétérocyclyle portant n groupes oxo, ou
R³ signifie phényle, qui est substitué par p radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆), alcoxy en (C₁-C₆)-alkyle en (C₁-C₄),
R⁴ signifie alkyle en (C₁-C₆), cycloalkyle en (C₃-C₇), halogéno-alkyle en (C₁-C₆), alcoxy en (C₁-C₆), halogéno-alcoxy en (C₁-C₆), cyano, nitro, méthylsulfényle, méthylsulfinyle, méthylsulfonyle, alkylcarbonylamino en (C₁-C₄), benzoylamino, méthoxycarbonyle, éthoxycarbonyle, benzoyle, phénoxy, méthylcarbonyle, pipéridinylcarbonyle, trifluorométhylcarbonyle, halogène, amino, aminocarbonyle, méthylaminocarbonyle, diméthylaminocarbonyle, méthoxyméthyle, 1,2,4-triazol-1-yle, pyrazol-1-yle, 2-thiophényle, 2-pyridinyle, 3-pyridinyle, 4-pyridinyle, 1,2,4-oxadiazol-3-yle, benzoxazol-2-yle, 1-éthylbenzimidaol-2-yle, pipéridin-1-yle, ou phényle, à chaque fois substitué par p radicaux du groupe constitué par méthyle, éthyle, méthoxy, trifluorométhyle et halogène,
R⁵ signifie hydrogène, alkyle en (C₁-C₆), cycloalkyle en (C₃-C₇), halogéno-alkyle en (C₁-C₆), cycloalkylméthyle en (C₃-C₇), méthoxycarbonylméthyle, éthoxycarbonylméthyle, acétylméthyle, méthoxyméthyle, méthoxyéthyle, benzyle, pyrazin-2-yle, furan-2-yle, tétrahydrofuran-2-yle, morpholine, diméthylamino, ou phényle substitué par p radicaux du groupe constitué par méthyle, méthoxy, trifluorométhyle et halogène,
R⁶ signifie halogène, cyano, hydroxy, amino, nitro,-CHO, -C(=O)OH, -C(=O)NH₂, -C(=S)NH₂, -C(=O)NHCN,-C(=O)NHOH, -SH, -SO₂NH₂, -SO₂NHCN, -SO₂NHOH, -OCN, -SCN, -SF₅, alkyle en (C₁-C₆), alcényle en (C₂-C₆), alcynyle en (C₂-C₆), halogéno-alkyle en (C₁-C₆), halogéno-alcényle en (C₂-C₆), halogéno-alcynyle en (C₂-C₆), cycloalkyle en (C₃-C₈), halogéno-cycloalkyle en (C₃-C₈), alkyle en (C₁-C₆)-cycloalkyle en (C₃-C₇), cycloalkyle en (C₃-C₇)-alkyle en (C₁-C₆), cycloalcényle en (C₃-C₈), halogéno-cycloalcényle en (C₃-C₈), alcoxy en (C₁-C₆)-alkyle en (C₁-C₆), cycloalcoxy en (C₃-C₇)-alkyle en (C₁-C₆), alcoxy en (C₁-C₆)-alcoxy en (C₁-C₆)-alkyle en (C₁-C₆), alkylthio en (C₁-C₆)-alkyle en (C₁-C₆), alkylsulfinyle en (C₁-C₈)-alkyle en (C₁-C₆), alcoxy en (C₁-C₈)-halogéno-alkyle en (C₁-C₆), cyano-alkyle en (C₁-C₆), hydroxy-alkyle en (C₁-C₆), alcoxy en (C₁-C₆), halogéno-alcoxy en (C₁-C₆),
cycloalcoxy en (C₃-C₈), halogéno-cycloalcoxy en (C₃-C₈), cycloalkyle en (C₃-C₈)-alcoxy en (C₁-C₆), alcényloxy en (C₂-C₆), halogéno-alcényloxy en (C₂-C₆), alcoxy en (C₁-C₆)-alcoxy en (C₁-C₆), alkylcarbonyloxy en (C₂-C₆), alkylthio en (C₁-C₆), halogéno-alkylthio en (C₁-C₆), cycloalkylthio en (C₃-C₈), alkylsulfinyle en (C₁-C₆), halogéno-alkylsulfinyle en (C₁-C₆), alkylsulfonyle en (C₁-C₆), halogéno-alkylsulfonyle en (C₁-C₆), cycloalkylsulfonyle en (C₃-C₈), alkylamino en (C₁-C₆), di-alkylamino en (C₁-C₆), halogéno-alkylamino en (C₁-C₆),
halogéno-di-alkylamino en (C₁-C₈) ou cycloalkylamino en (C₃-C₈), ou deux radicaux R⁶ vicinaux forment un groupe alkylène en (C₃-C₆), dans lequel p atomes de carbone sont remplacés par oxygène, soufre ou azote, et dans lequel un atome de carbone porte t groupes oxo,
Y et Z signifient chacun indépendamment l'un de l'autre 0 ou S,
W¹ signifie alkylène en (C₁-C₁₀), alcénylène en (C₂-C₆) ou alcynylène en (C₂-C₆),
W² signifie alkylène en (C₁-C₁₀),
G signifie hétéroaryle ou hétérocyclyle, chacun substitué par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆) et alcoxy en (C₁-C₆)-alkyle en (C₁-C₄), hétérocyclyle portant n groupes oxo,
n signifie 0, 1 ou 2,
p signifie 0, 1, 2 ou 3,
s signifie 0, 1, 2, 3, 4 ou 5,
t signifie 0 ou 1.

3. 1,2,4-Triazine-3,5-dione-6-carboxamides de formule (I) ou leurs sels selon la revendication 1 ou 2, dans lesquels
R¹ signifie alkyle en (C₁-C₁₀), alcényle en (C₂-C₁₀), alcynyle en (C₂-C₁₀), halogéno-alkyle en (C₁-C₁₀), halogéno-alcényle en (C₂-C₁₀), halogéno-alcynyle en (C₂-C₁₀), cycloalkyle en (C₃-C₁₀), halogéno-cycloalkyle en (C₃-C₁₀), alkyle en (C₁-C₄)-cycloalkyle en (C₃-C₇), cycloalkyle en (C₃-C₇)-alkyle en (C₁-C₆), cycloalkyle en (C₃-C₇)-cycloalkyle en (C₃-C₇), halogéno-cycloalkyle en (C₃-C₇)-alkyle en (C₁-C₆), alkyle en (C₁-C₆)-cycloalkyle en (C₃-C₇)-alkyle en (C₁-C₆), cycloalcényle en (C₃-C₁₂), halogéno-cycloalcényle en (C₃-C₁₂), alcoxy en (C₁-C₆)-alkyle en (C₁-C₆), alcoxy en (C₁-C₆)-alcényle en (C₂-C₆), alkyle en (C₁-C₆)-cycloalkyle en (C₃-C₇), alcoxy en (C₁-C₆)-cycloalkyle en (C₃-C₇), cycloalcoxy en (C₃-C₇)-alkyle en (C₁-C₆), cycloalcoxy en (C₃-C₇)-alcoxy en (C₁-C₆)-alkyle en (C₁-C₆), alcoxy en (C₁-C₆)-alcoxy en (C₁-C₆)-alkyle en (C₁-C₆), alkylthio en (C₁-C₆)-alkyle en (C₁-C₆), alkylsulfinyle en (C₁-C₆)-alkyle en (C₁-C₆), alkylsulfonyle en (C₁-C₆)-alkyle en (C₁-C₆), alkylamino en (C₁-C₆)-alkyle en (C₁-C₆), di-alkylamino en (C₁-C₆)-alkyle en (C₁-C₆), halogéno-alkylamino en (C₁-C₆)-alkyle en (C₁-C₆), cycloalkylamino en (C₃-C₇)-alkyle en (C₁-C₆), alkyle en (C₁-C₆)-carbonyle, halogéno-alkyle en (C₁-C₆)-carbonyle, cycloalkyle en (C₃₋-C₇)-carbonyle, alcoxy en (C₁-C₆)-carbonyle, cycloalcoxy en (C₃-C₇)-carbonyle, cycloalkyle en (C₃-C₇)-alcoxy en (C₁-C₆)-carbonyle, alkylamino en (C₁-C₆)-carbonyle, di-alkylamino en (C₁-C₆)-carbonyle, cycloalkylamino en (C₃-C₇)-carbonyle, cyano-alkyle en (C₁-C₆), hydroxy-alkyle en (C₁-C₆), cycloalcényle en (C₃-C₇)-alkyle en (C₁-C₆), halogéno-alcoxy en (C₁-C₆)-alkyle en (C₁-C₆), alcoxy en (C₁-C₆)-halogéno-alkyle en (C₁-C₆), halogéno-alcoxy en (C₁-C₆)-halogéno-alkyle en (C₁-C₆), halogéno-cycloalcoxy en (C₃-C₇)-alkyle en (C₁-C₆), cycloalcényloxy en (C₃-C₇)-alkyle en (C₁-C₆), halogéno-cycloalcényloxy en (C₃-C₇)-alkyle en (C₁-C₆), di-alcoxy en (C₁-C₆)-alkyle en (C₁-C₆), alcoxy en (C₁-C₆)-alkyle en (C₁-C₆)-carbonyle, alcoxy en (C₁-C₆)-carbonyl-alkyle en (C₁-C₆), halogéno-alcoxy en (C₁-C₆)-carbonyle, alcoxy en (C₁-C₁₀), halogéno-alcoxy en (C₁-C₁₀), cycloalcoxy en (C₃-C₁₂), halogéno-cycloalcoxy en (C₃-C₇), cycloalkyle en (C₃-C₇)-alcoxy en (C₁-C₆), alcényloxy en (C₂-C₁₂), halogéno-alcényloxy en (C₂-C₁₀), alcynyloxy en (C₂-C₁₀), halogéno-alcynyloxy en (C₃-C₁₀), alcoxy en (C₁-C₆)-alcoxy en (C₁-C₆), alkyle en (C₁-C₆)-carbonyloxy, halogéno-alkyle en (C₂-C₁₂)-carbonyloxy, cycloalkyle en (C₃-C₇)-carbonyloxy, alkyle en (C₁-C₆)-carbonyl-alcoxy en (C₁-C₆), alkylthio en (C₁-C₁₀), halogéno-alkylthio en (C₁-C₁₀), cycloalkylthio en (C₃-C₁₂), alkylsulfinyle en (C₁-C₁₀), halogéno-alkylsulfinyle en (C₁-C₁₀), alkylsulfonyle en (C₁-C₁₀), halogéno-alkylsulfonyle en (C₁-C₁₀), cycloalkylsulfonyle en (C₃-C₁₂), alkyle en (C₁-C₆)-carbonylthio, alkyle en (C₁-C₆)-(thiocarbonyl)thio, cycloalkylsulfinyle en (C₃-C₁₂), alkylaminosulfonyle en (C₁-C₆), di-alkylamino en (C₁-C₆)-sulfonyle, alkylamino en (C₁-C₆), di-alkylamino en (C₁-C₁), halogéno-alkylamino en (C₁-C₆), halogéno-di-alkylamino en (C₁-C₆), cycloalkylamino en (C₃-C₁₂), alkyle en (C₁-C₆)-carbonylamino, halogéno-alkyle en (C₁-C₆)-carbonylamino, alkylsulfonylamino en (C₁-C₁₀), halogéno-alkylsulfonylamino en (C₁-C₁₀), cycloalkyle en (C₃-C₇)-alkyle en (C₁-C₆)-amino, cyano, hydroxy, amino, C(=O)OH, C(=O)NHOH, SO₂NH₂, SO₂NHCN, SO₂NHOH, NHCHO ou
R¹ signifie phényle, W¹-(phényle), les cycles phényle portant chacun s substituants R⁶, ou
R¹ signifie G,
R² signifie alkyle en (C₁-C₄), haloalkyle en (C₁-C₄), phényle, hétéroaryle, les cycles phényle ou hétéroaryle des deux radicaux susmentionnés portant à chaque fois s substituants R⁶,
Q signifie un radical Q¹, Q², Q³ ou Q⁴ 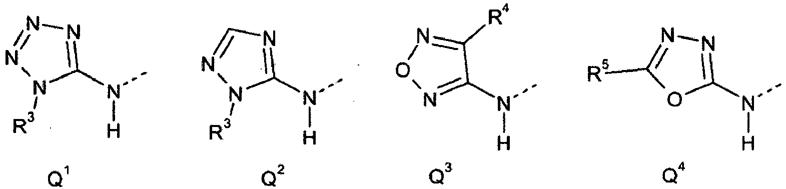 R³ signifie alkyle en (C₁-C₈), alcényle en (C₂-C₈) ou alcynyle en (C₂-C₈), chacun substitués par s radicaux du groupe constitué par halogène, cyano, nitro, S(O)ₙ-alkyle en (C₁-C₆), alcoxy en (C₁-C₆) et halogéno-alcoxy en (C₁-C₆),
R⁴ signifie alkyle en (C₁-C₆), cycloalkyle en (C₃-C₇), halogéno-alkyle en (C₁-C₆), alcoxy en (C₁-C₆), halogéno-alcoxy en (C₁-C₆), cyano, nitro, méthylsulfényle, méthylsulfinyle, méthylsulfonyle, alkylcarbonylamino en (C₁-C₄), benzoylamino, méthoxycarbonyle, éthoxycarbonyle, benzoyle, phénoxy, méthylcarbonyle, pipéridinylcarbonyle, trifluorométhylcarbonyle, halogène, amino, aminocarbonyle, méthylaminocarbonyle, diméthylaminocarbonyle, méthoxyméthyle, 1,2,4-triazol-1H, 1-pyrazol-1H, 2-thiophényle, 2-pyridinyle, 3-pyridinyle, 4-pyridinyle, 1,2,4-oxadiazol-3-yle, benzoxazol-2-yle, 1-éthylbenzimidaol-2-yle, pipéridin-1-yle, ou phényle, à chaque fois substitué par s radicaux du groupe constitué par méthyle, éthyle, méthoxy, trifluorométhyle et halogène,
R⁵ signifie hydrogène, alkyle en (C₁-C₆), cycloalkyle en (C₃-C₇), halogéno-alkyle en (C₁-C₆), cycloalkylméthyle en (C₃-C₇), méthoxycarbonylméthyle, éthoxycarbonylméthyle, acétylméthyle, méthoxyméthyle, méthoxyéthyle, benzyle, pyrazin-2-yle, furan-2-yle, tétrahydrofuran-2-yle, morpholine, diméthylamino, ou phényle substitué par p radicaux du groupe constitué par méthyle, méthoxy, trifluorométhyle et halogène,
R⁶ signifie halogène, cyano, nitro, alkyle en (C₁-C₆), alcényle en (C₂-C₆), alcynyle en (C₂-C₆), halogéno-alkyle en (C₁-C₆), cycloalkyle en (C₃-C₆), alcoxy en (C₁-C₆), halogéno-alcoxy en (C₁-C₆), alkylthio en (C₁-C₆), alkylsulfinyle en (C₁-C₆), alkylsulfonyle en (C₁-C₆), phényle, méthylènedioxo,
Y et Z signifient chacun indépendamment l'un de l'autre 0 ou S,
W¹ signifie alkylène en (C₁-C₁₀),
G signifie hétéroaryle ou hétérocyclyle, chacun substitué par s radicaux du groupe constitué par halogène, nitro, cyano, alkyle en (C₁-C₆), halogéno-alkyle en (C₁-C₃), cyclopropyle, S(O)ₙ-alkyle en (C₁-C₃), alcoxy en (C₁-C₉), halogéno-alcoxy en (C₁-C₄) et alcoxy en (C₁-C₄)-alkyle en (C₁-C₄), hétérocyclyle portant n groupes oxo,
n signifie 0, 1 ou 2,
p signifie 0, 1, 2 ou 3,
s signifie 0, 1, 2, 3, 4 ou 5.

4. Agent herbicide, **caractérisé par** une teneur herbicide efficace en au moins un composé de formule (I) selon l'une quelconque des revendications 1 à 3.

5. Agent herbicide selon la revendication 4, en mélange avec des adjuvants de formulation.

6. Agent herbicide selon la revendication 4 ou 5, contenant au moins un agent actif pesticide supplémentaire du groupe constitué par les insecticides, les acaricides, les herbicides, les fongicides, les agents protecteurs et les régulateurs de croissance.

7. Agent herbicide selon la revendication 6, contenant un agent protecteur.

8. Agent herbicide selon la revendication 7, contenant du cyprosulfamide, du cloquintocet-mexyl, du méfenpyr-diéthyle ou de l'isoxadifène-éthyle.

9. Agent herbicide selon l'une quelconque des revendications 4 à 8, contenant un herbicide supplémentaire.

10. Procédé de lutte contre des plantes indésirables, **caractérisé en ce qu'**une quantité efficace d'au moins un composé de formule (I) selon l'une quelconque des revendications 1 à 3 ou d'un agent herbicide selon l'une quelconque des revendications 4 à 8 est appliquée sur les plantes ou à l'emplacement de la végétation indésirable.

11. Utilisation de composés de formule (I) selon l'une quelconque des revendications 1 à 3 ou d'agents herbicides selon l'une quelconque des revendications 4 à 8 pour lutter contre des plantes indésirables.

12. Utilisation selon la revendication 11, **caractérisée en ce que** les composés de formule (I) sont utilisés pour lutter contre des plantes indésirables dans des cultures de plantes utiles.

13. Utilisation selon la revendication 12, **caractérisée en ce que** les plantes utiles sont des plantes utiles transgéniques.
